# EUROPEAN PATENT SPECIFICATION

(11) **EP 3 849 533 B1**
(45) Date of publication and mention of the grant of the patent: **28.10.2026**
(21) Application number: 19860696.4
(22) Date of filing: 13.09.2019
(51) Int. Cl.: A61K 31/137, A61K 31/195, A61P 27/10, A61P 27/02, A61K 9/08, A61K 47/22

(54) **TOPICAL COMPOSITION COMPRISING DOPAMINE OR DEUTERATED DOPAMINE FOR USE IN THE TREATMENT OF AN OCULAR DISEASE**
TOPISCHE ZUSAMMENSETZUNG ENTHALTEND DOPAMIN ODER DEUTERIERTES DOPAMIN ZUR ANWENDUNG IN DER BEHANDLUNG EINER AUGENERKRANKUNG
COMPOSITION TOPIQUE COMPRENANT DE LA DOPAMINE OU DE LA DOPAMINE DEUTÉRÉE DESTINÉE À ÊTRE UTILISÉE DANS LE TRAITEMENT D'UNE MALADIE OCULAIRE

(30) Priority: 13.09.2018 AU 2018903445
(43) Date of publication of application: 21.07.2021
(73) Proprietor: University of Canberra, Bruce, Australian Capital Territory 2617 (AU)
(72) Inventor: ASHBY, Regan Scott, Franklin, Australian Capital Territory 2913 (AU)
(74) Representative: Vossius & Partner Patentanwälte Rechtsanwälte mbB
(86) International application number: PCT/AU2019/050986
(87) International publication number: WO 2020/051648

(56) References cited:
- WO-A1-2016/187426
- WO-A1-2016/187426
- WO-A1-2017/060870
- WO-A1-2017/177262
- WO-A1-2017/177262
- WO-A2-2015/095257
- US-A1- 2016 009 705
- US-A1- 2018 002 700
- QIANYING GAO ET AL: "Effects of direct intravitreal dopamine injections on the development of lid-suture induced myopia in rabbits", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 244, no. 10, 21 March 2006 (2006-03-21), pages 1329 - 1335, XP019443108, ISSN: 1435-702X, DOI: 10.1007/S00417-006-0254-1
- MALMLOF T ET AL: "Altered behavioural and neurochemical profile of l-DOPA following deuterium substitutions in the molecule", EXPERIMENTAL NEUROLOGY, ELSEVIER, AMSTERDAM, NL, vol. 212, no. 2, 1 August 2008 (2008-08-01), pages 538 - 542, XP023179561, ISSN: 0014-4886, [retrieved on 20080724], DOI: 10.1016/J.EXPNEUROL.2008.05.003
- SHANNON , R. ET AL.: "The effect of dopamine on the intraocular pressure and pupil of the rabbit eye", INVESTIGATIVE OPHTHALMOLOGY, vol. 15, no. 5, 1976, pages 371 - 380, XP008071614, Retrieved from the Internet <URL:https://iovs.arvojournals.org/data/Journals/IOVS/933300/371.pdf> [retrieved on 20190711]
- GAO, Q. ET AL.: "Effects of direct intravitreal dopamine injections on the development of lid-suture induced myopia in rabbits", GRAEFE'S ARCHIVE FOR CLINICAL & EXPERIMENTAL OPHTHALMOLOGY, vol. 244, no. 10, 2006, pages 1329 - 1335, XP019443108, [retrieved on 20190708], DOI: 10.1007/s00417-006-0254-1
- LIN, Z. ET AL.: "Effects of Direct Intravitreal Dopamine Injection on Sclera and Retina in Form-Deprived Myopic Rabbits", JOURNAL OF OCULAR PHARMACOLOGY AND THERAPEUTICS, vol. 24, no. 6, 2008, pages 543 - 550, XP055692563, [retrieved on 20190708]

## Description

This description relates generally to the use of dopamine, deuterated dopamine, a deuterated dopamine derivative, or a pharmaceutically acceptable salt thereof for inhibiting the development or progression of a visual disorder, such as myopia. The present invention relates to a composition comprising dopamine or a pharmaceutically acceptable salt thereof, or deuterated dopamine or a pharmaceutically acceptable salt thereof, and an aqueous carrier for use in inhibiting the development or progression of a visual disorder of the posterior segment of the eye in a subject, wherein said composition is to be topically administered to an eye of the subject, and wherein said composition is formulated for topical administration to an eye of the subject.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavor to which this specification relates.

Myopia, commonly known as short-sightedness, is a visual disorder caused by excessive elongation (axial length) of the eye during development. Myopia is the leading cause of low vision and the most common eye disease worldwide, with some estimating that myopia may affect up to one-third of the world's population by the end of the decade. Prevalence is at its highest in urban East Asia, where in many parts approximately 80-90% of school leavers are myopic.

The prevalence of myopia appears to be strongly associated with the amount of time spent outdoors in bright light. Specifically, epidemiological studies have reported that time spent outdoors is a potent protective factor against the development of myopia in children. Animal studies have indicated that this protective effect appears to be associated with light induced increases in dopamine levels within the eye.

Attempts are being made to reduce the onset and progression of myopia, including increasing the amount of time that children spend outdoors in bright light. However, in many parts of the world geographical location and local climate restrictions may prevent light levels from being strong enough or exposure time from being long enough to protect against myopia. Furthermore, social and cultural barriers may prevent increasing the time children spend outdoors as it is perceived as hindering education and academic progression.

Current treatment options to reduce the progression of myopia include optical approaches, such as single vision lenses, multifocal lenses, peripheral lenses and orthokeratology; and pharmaceutical agents, such as atropine and pirenzepine. With regards to optical approaches, findings from clinical trials have been mixed, with the majority of optical approaches showing limited to no long-term effect on the rate of myopia progression. Optical approaches are also not targeted at preventing the onset of myopia, only its progression. Traditionally, treatment with pharmaceutical agents, such as atropine, have been most effective at reducing the rate of myopia progression. However, the widespread use of atropine has been inhibited by concerns about post-treatment rebound effects, as well as the significant short- and long-term adverse effects.

Drug delivery to the posterior segment of the eye poses a significant challenge due to the multitude of barriers present in the eye. This is particularly important for topically delivered therapeutics, where it is estimated that less than 5% of a topically administered drug reaches intraocular tissues (Janoria et al. (2007) Expert Opin Drug Deliv, 4(4): 371-88; Mantelli et al. (2013) Curr Opin Allergy Clin Immunol, 13(5): 563-568). Problems with topical administration for delivery of drugs to the posterior segment of the eye include extensive precorneal drug loss by high tear fluid turnover, non-productive absorption, drainage through the nasolacrimal duct, impermeability of the corneal epithelium, transient precorneal residence time and metabolism of the drug by anterior segment enzymes (Janoria et al. (2007) Expert Opin Drug Deliv, 4(4): 371-88). One of the main barriers to drug penetration into the eye is the corneal epithelium. The corneal epithelium is similar in structure to the blood brain barrier, with tight junctions surrounding the cells below the apical surface (Mantelli et al. (2013) Curr Opin Allergy Clin Immunol, 13(5): 563-568). Similarly to the blood brain barrier, the tight junctions in the corneal epithelium are primarily responsible for the barrier to entry of pathogens and topically administered drugs (Mantelli et al. (2013) Curr Opin Allergy Clin Immunol, 13(5): 563-568). Intravitreal injection is tested, however, is not considered a good therapeutic way to treat myopia (Gao et al. (2006) , Graefe's Arch Clin Exp Ophthalmol 244:1329-1335*).* Therefore, drugs which can overcome these barriers are advantageous as therapies for ocular disorders, for example levodopa (WO 2017/177262).

New therapies for inhibiting the development or progression of a visual disorder, such as myopia, are required.

The present invention relates to the embodiments as set forth in the amended claims. Particularly, the present invention relates to a composition comprising dopamine or a pharmaceutically acceptable salt thereof, or deuterated dopamine or a pharmaceutically acceptable salt thereof, and an aqueous carrier for use in inhibiting the development or progression of a visual disorder of the posterior segment of the eye in a subject, wherein said composition is to be topically administered to an eye of the subject, and wherein said composition is formulated for topical administration to an eye of the subject.

In the present disclosure, reference to "methods of treatment, use, administration" and analogous terms comprising administration of one or more active agents, compositions, pharmaceutical compositions, etc., are not to be understood as methods practiced on the human or animal body but are directed to the active agents, compositions, pharmaceutical compositions, etc., for use in the disclosed treatment.

The term "visual disorder" is frequently used in the present disclosure. In context of the claimed invention it is understood that "visual disorder" refers to a "visual disorder of the posterior segment of the eye".

The present invention is predicated in part on the discovery that dopamine [2-(3,4-dihydroxyphenyl)ethylamine] or a deuterated dopamine can penetrate ocular tissues and affect structures in the posterior segment of the eye, including the retina. In view of the inability of dopamine to cross the blood brain barrier, it was not thought that dopamine, or deuterated dopamine would be able to cross the corneal epithelium due to structural similarities with the blood brain barrier. Surprisingly, the inventors have found that dopamine and deuterated dopamine are able to penetrate the corneal epithelium and affect structures in the posterior segment of the eye. Accordingly, the present inventors conceived that dopamine, or a deuterated dopamine can be locally administered to an eye of a subject to inhibit the development or progression of a visual disorder in the subject, particularly a visual disorder in the posterior segment of the eye involving reduced dopamine levels, such as myopia, a visual disorder associated with diabetic retinopathy or a visual disorder associated with Parkinson's disease.

In one aspect of the invention, there is provided a method for inhibiting the progression or development of a visual disorder in a subject, comprising topically administering a composition comprising dopamine or a pharmaceutically acceptable salt thereof to an eye of the subject. In some embodiments, the composition is topically administered to both eyes of the subject.

In a further aspect, there is provided a use of a composition comprising dopamine or a pharmaceutically acceptable salt thereof for inhibiting the development or progression of a visual disorder in a subject, wherein the composition is topically administered to an eye of the subject.

In yet another aspect of the invention, there is provided a composition comprising dopamine or a pharmaceutically acceptable salt thereof for use in inhibiting the development or progression of a visual disorder in a subject, wherein the composition is formulated for topical administration to an eye of the subject.

The present invention also provides a use of a composition comprising dopamine or a pharmaceutically acceptable salt thereof in the manufacture of a medicament for inhibiting the development or progression of a visual disorder in a subject, wherein the composition is formulated for topical administration to an eye of the subject.

In another aspect of the invention, there is provided a method for inhibiting the development or progression of a visual disorder in a subject, comprising locally administering a composition comprising a deuterated dopamine, or a pharmaceutically acceptable salt thereof to an eye of the subject. In some embodiments, the composition is administered to both eyes of the subject.

In a further aspect, there is provided a use of a composition comprising a deuterated dopamine or a pharmaceutically acceptable salt thereof for inhibiting the development or progression of a visual disorder in a subject, wherein the composition is locally administered to an eye of the subject.

In another aspect, there is provided a composition comprising a deuterated dopamine or a pharmaceutically acceptable salt thereof for use in inhibiting the development or progression of a visual disorder in a subject, wherein the composition is formulated for local administration to an eye of the subject.

In still another aspect, there is provided a use of a composition comprising a deuterated dopamine, or a pharmaceutically acceptable salt thereof in the manufacture of a medicament for inhibiting the development or progression of a visual disorder in a subject, wherein the composition is formulated for local administration to an eye of the subject.

In particular embodiments of any one of the above aspects, the deuterated dopamine or pharmaceutically acceptable salt thereof is a compound of Formula I: 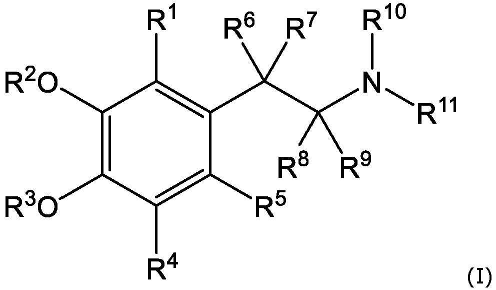
or a pharmaceutically acceptable salt thereof, wherein
R¹, R², R³, R⁴, R⁵, R⁶, R⁷, R⁸, R¹⁰ and R¹¹ are each independently selected from H and D;
R⁹ is selected from H and D; and
wherein at least one of R¹ to R¹¹ is D.

### DRAWINGS

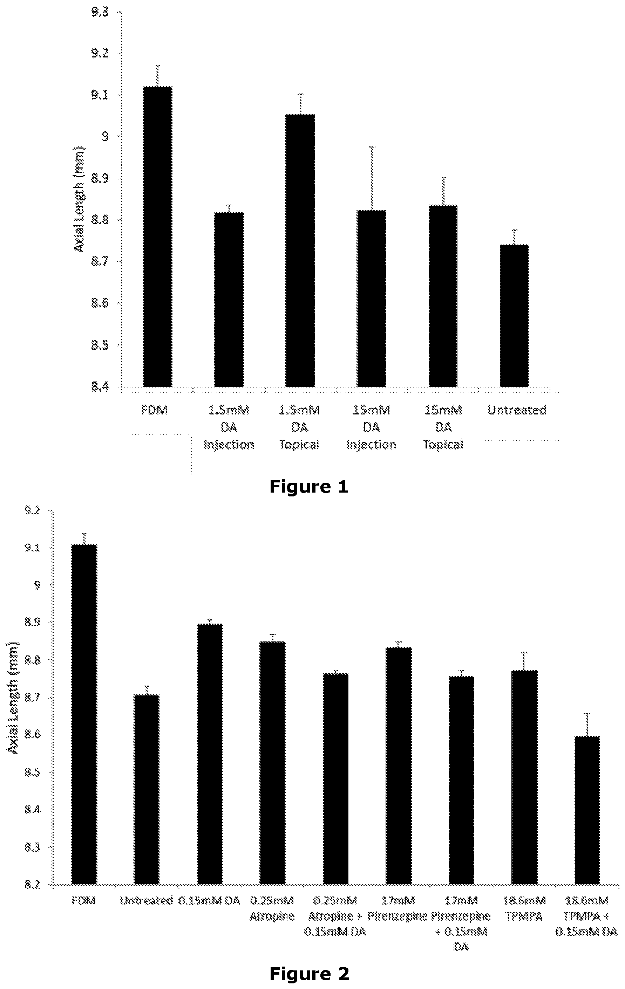

Figure 1 shows the average axial length (mm) in chick eyes in response to diffuser-wear (FDM), and intravitreal injection (injection) and topical administration (topical) of dopamine (DA) compositions compared to untreated, age-matched controls. Error bars represent the standard error of the mean.
Figure 2 shows the average axial length (mm) in chick eyes in response to diffuser-wear (FDM), and intravitreal injection of dopamine (DA), atropine, pirenzepine, TPMPA, and dopamine in combination with atropine, pirenzepine and TPMPA compared to untreated, age-matched controls. Error bars represent the standard error of the mean.

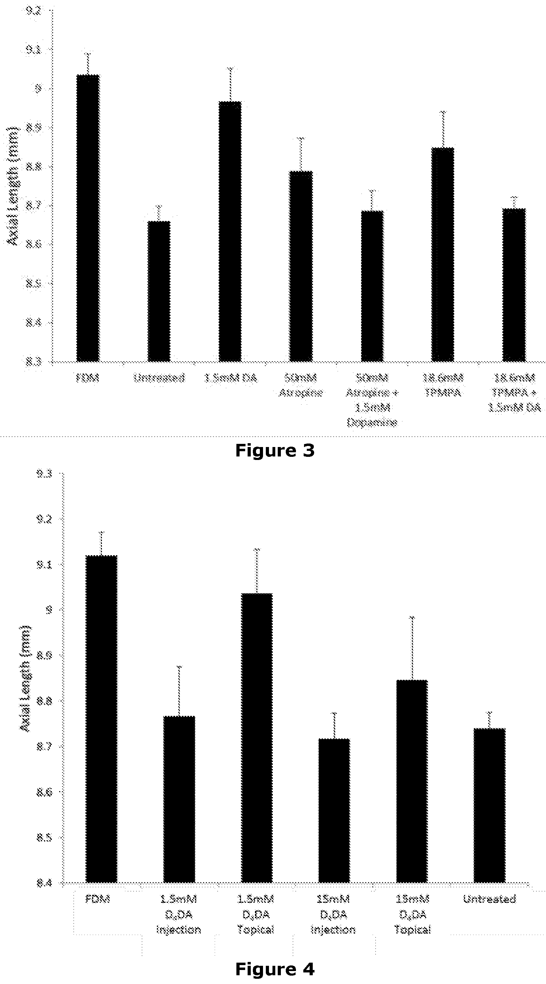

Figure 3 shows the average axial length (mm) in chick eyes in response to diffuser-wear (FDM), and topical administration of dopamine (DA), atropine, TPMPA, and dopamine in combination with atropine and TPMPA compared to untreated, age-matched controls. Error bars represent the standard error of the mean.
Figure 4 shows the average axial length (mm) in chick eyes in response to diffuser-wear, and intravitreal injection (injection) and topical administration (topical) of dopamine-1,1,2,2-d₄ (D₄DA) compositions compared to untreated, age-matched controls. Error bars represent the standard error of the mean.

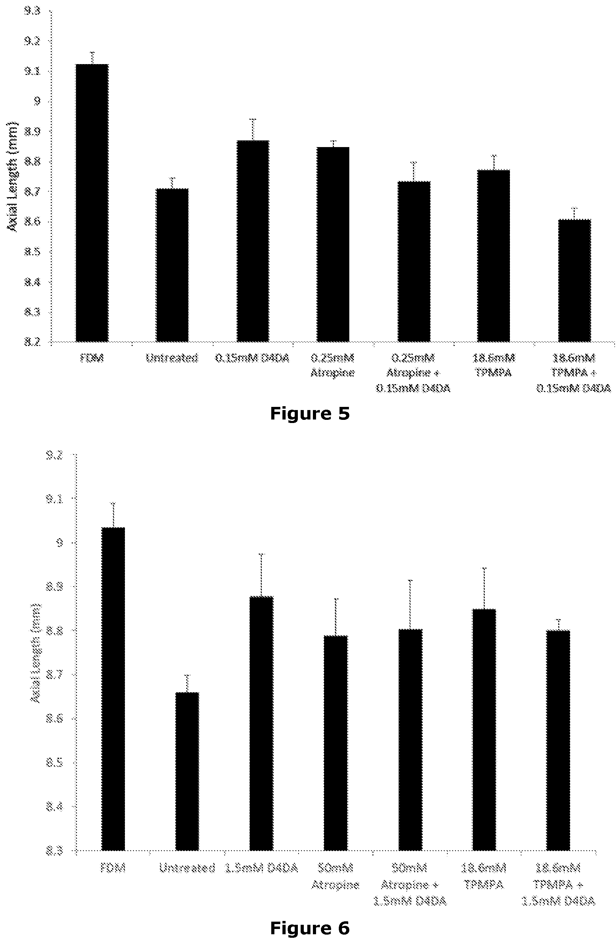

Figure 5 shows the average axial length (mm) in chick eyes in response to diffuser-wear (FDM), and intravitreal injection of dopamine-1,1,2,2-d₄ (D4DA), atropine, TPMPA, and dopamine-1,1,2,2-d₄ in combination with atropine and TPMPA compared to untreated, age-matched controls. Error bars represent the standard error of the mean.
Figure 6 shows the average axial length (mm) in chick eyes in response to diffuser-wear (FDM), and topical administration of dopamine-1,1,2,2-d₄ (D4DA), atropine, TPMPA, and dopamine-1,1,2,2-d₄ in combination with atropine and TPMPA compared to untreated, age-matched controls. Error bars represent the standard error of the mean.

### DETAILED DESCRIPTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "an element" means one element or more than one element.

By "about" is meant a quantity, level, value, number, frequency, percentage, dimension, size, amount, weight or length that varies by as much 15, 14, 13, 12, 11, 10, 9, 8, 7, 6, 5, 4, 3, 2 or 1 % to a reference quantity, level, value, number, frequency, percentage, dimension, size, amount, weight or length.

As used herein, the term "and/or" refers to and encompasses any and all possible combinations of one or more of the associated listed items, as well as the lack of combinations when interpreted in the alternative (or).

The term "carrier" is used herein to refer to a liquid diluent. By "pharmaceutically acceptable carrier" is meant a pharmaceutical vehicle comprised of a material that is not biologically or otherwise undesirable, i.e., the material may be administered to a subject along with the selected active agent without causing any or a substantial adverse reaction. Carriers may include excipients and other additives such as diluents, detergents, coloring agents, wetting or emulsifying agents, pH buffering agents, preservatives, and the like. In particular embodiments the carrier is an aqueous carrier. The term "aqueous carrier" is used herein to refer to a liquid aqueous diluent, wherein the aqueous carrier includes, but is not limited to, water, saline, aqueous buffer and aqueous solutions comprising water soluble or water miscible additives such as glucose or glycerol. The aqueous carrier may also be in the form of an oil-in-water emulsion.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. Thus, the use of the term "comprising" and the like indicates that the listed integers are required or mandatory, but that other integers are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

As used herein, the term "condition" refers to an abnormality in the physical state of the body as a whole or one of its parts.

The term "deuterated dopamine" is used herein to refer to dopamine comprising at least one deuterium atom in place of a hydrogen atom. For example, "deuterated dopamine" may refer to dopamine comprising at least 1, 2, 3, 4, 5, 6, 7, 8, 9, 10 or 11 deuterium atoms.

As used herein, the terms "salts" and "prodrugs" include any pharmaceutically acceptable salt, ester, hydrate, solvate or any other compound which, upon administration to the recipient, is capable of providing (directly or indirectly) a desired compound, or an active metabolite or residue thereof. Suitable pharmaceutically acceptable salts include salts of pharmaceutically acceptable inorganic acids such as hydrochloric, sulfuric, phosphoric, nitric, carbonic, boric, sulfamic and hydrobromic acids, or salts of pharmaceutically acceptable organic acids such as acetic, propionic, butyric, tartaric, maleic, hydroxymaleic, fumaric, citric, lactic, mucic, gluconic, benzoic, succinic, oxalic, phenylacetic, methanesulfonic, toluenesulfonic, benzenesulfonic, salicylic, sulfanilic, aspartic, glutamic, edetic, stearic, palmitic, oleic, lauric, pantothenic, tannic, ascorbic and valeric acids. Base salts include, but are not limited to, those formed with pharmaceutically acceptable cations, such as sodium, potassium, lithium, calcium, magnesium, ammonium and alkylammonium. Also, basic nitrogen-containing groups may be quaternized with such agents as lower alkyl halides, such as methyl, ethyl, propyl and butyl chlorides, bromides and iodides; dialkyl sulfates such as dimethyl and diethyl sulfate; and others. However, it will be appreciated that non-pharmaceutically acceptable salts also fall within the scope of the invention since these may be useful in the preparation of pharmaceutically acceptable salts. The preparation of salts and prodrugs can be carried out by methods known in the art. For example, metal salts can be prepared by reaction of a desired compound with a metal hydroxide. An acid salt can be prepared by reacting an appropriate acid with a desired compound.

As used herein, the phrase "solubilized form" refers to a form where a compound, such as dopamine or a deuterated dopamine, is dissolved in a liquid such that a solution comprising a uniform distribution of the compound is obtained which is substantially free of solid compound. In some embodiments, the liquid is an aqueous carrier as described herein.

The term "subject" as used herein refers to a vertebrate subject, particularly a mammalian or avian subject, for whom therapy or prophylaxis is desired. Suitable subjects include, but are not limited to, primates; birds; livestock animals such as sheep, cows, horses, deer, donkeys and pigs; laboratory test animals such as rabbits, mice, rats, guinea pigs and hamsters; companion animals such as cats and dogs; and captive wild animals such as foxes, deer and dingoes. In particular embodiments, the subject is a human. In some embodiments, the subject is a human child or young adult, for example, from the age of about 2 years to 20 years. However, it will be understood that the aforementioned terms do not imply that symptoms are present.

As used herein, the phrase "visual disorder" refers to a condition that alters the vision of a subject. In particular embodiments, such conditions are associated with a decrease in "visual acuity", which is typically associated with diminishing or lessening of the acuteness or clearness of vision. Thus, a decrease in "visual acuity" typically refers to any measurable diminishing or lessening in the acuteness or clearness of form vision, which is dependent on the sharpness of the retinal focus within the eye and the sensitivity of the interpretative faculty of the brain. In certain embodiments, visual acuity refers to the Snellen acuity (e.g. 20/20). The visual disorder may be a disease, disorder or condition.

Each embodiment described herein is to be applied *mutatis mutandis* to each and every embodiment unless specifically stated otherwise.

### 2. Abbreviations

The following abbreviations are used throughout the application:
D =deuterium

### 3. Compositions

The present invention is predicated in part on the discovery that dopamine [2-(3,4-dihydroxyphenyl)ethylamine] or a deuterated dopamine can penetrate ocular tissues and affect structures in the posterior segment of the eye, including the retina. Accordingly, the present inventors conceived that compositions comprising dopamine or a deuterated dopamine can be locally administered to inhibit the development or progression of a visual disorder in a subject, particularly a visual disorder in the posterior segment of the eye involving reduced dopamine levels, such as myopia, a visual disorder associated with diabetic retinopathy or a visual disorder associated with Parkinson's disease.

In one aspect of the invention, the composition comprises dopamine or a pharmaceutically acceptable salt thereof. In preferred embodiments, such composition is formulated for topical administration to an eye, such as in the form of an eye drop. In particular embodiments, the composition comprises dopamine or a pharmaceutically acceptable salt thereof and a pharmaceutically acceptable carrier. In some embodiments, the composition comprises dopamine or a pharmaceutically acceptable salt thereof, a pharmaceutically acceptable carrier and an antioxidant.

In another aspect of the invention, the composition comprises a deuterated dopamine, or a pharmaceutically acceptable salt thereof. Such compositions are formulated for local administration to an eye of the subject, as topical administration to an eye, such as in the form of an eye drop, or direct injection into an eye. In particular embodiments, the composition comprises a deuterated dopamine, or a pharmaceutically acceptable salt thereof, and a pharmaceutically acceptable carrier. In some embodiments, the composition comprises a deuterated dopamine, or a pharmaceutically acceptable salt thereof, a pharmaceutically acceptable carrier and an antioxidant.

In some embodiments, the composition comprises a deuterated dopamine or a pharmaceutically acceptable salt thereof. The deuterated dopamine may comprise one or more deuterium atoms in place of hydrogen. For example, the deuterated dopamine may comprise 1, 2, 3, 4, 5, 6, 7, 8, 9, 10 or 11 deuterium atoms; especially 2, 3 or 4 deuterium atoms; most especially 4 deuterium atoms. In particular embodiments, the deuterated dopamine is dopamine-1,1,2,2-d₄ [2-(3,4-dihydroxyphenyl)ethyl-1,1,2,2,d₄-amine]; 2-(3,4-dihydroxyphenyl)ethyl-1-deutero-amine; 2-(3,4-dihydroxyphenyl)ethyl-2,2-dideutero-amine; or a pharmaceutically acceptable salt thereof; especially dopamine-1,1,2,2-d₄ hydrochloride.

Also provided is a composition that comprises a deuterated dopamine derivative or a pharmaceutically acceptable salt thereof. The deuterated dopamine derivative may comprise one or more deuterium atoms in place of hydrogen. For example, the deuterated dopamine derivative may comprise 1, 2, 3, 4, 5, 6, 7, 8, 9, 10, 11 or 12 deuterium atoms; especially 2 or 3 deuterium atoms; most especially 3 deuterium atoms. , Also provided is a deuterated dopamine derivativewhich may be deuterated levodopa or a pharmaceutically acceptable salt thereof. The deuterated levodopa may be, but is not limited to, 2-amino-2-deutero-3-(3,4-dihydroxyphenyl) propionic acid; 2-amino-2,3-dideutero-3-(3,4-dihydroxyphenyl) propionic acid; 2-amino-2,3,3-trideutero-3-(3,4-dihydroxyphenyl) propionic acid; 2-amino-3,3-dideutero-3-(3,4-dihydroxyphenyl) propionic acid; 2-amino-3,3-dideutero-3-(3,4-dideuteroxyphenyl) propionic acid; 2-amino-2-deutero-3-(2,3,6-trideutero-4,5-dihydroxyphenyl) propionic acid; 2-amino-2,3-dideutero-3-(2,3,6-trideutero-4,5-dihydroxyphenyl) propionic acid; 2-amino-2,3,3-trideutero-3-(2,3,6-trideutero-4,5-dihydroxyphenyl) propionic acid; 2-amino-2,3,3-trideutero-3-(2,3,6-trideutero-4,5-dideuteroxyphenyl) propionic acid; or a pharmaceutically acceptable salt thereof; especially 2-amino-2,3-dideutero-3-(3,4-dihydroxyphenyl) propionic acid; 2-amino-2,3,3-trideutero-3-(3,4-dihydroxyphenyl) propionic acid; or a pharmaceutically acceptable salt thereof. The deuterated dopamine derivative or pharmaceutically acceptable salt thereof may be selected from the compounds disclosed in WO 2004/056724 A1, WO 2007/093450 A1, and WO 2014/122184 A1.

In particular embodiments, the deuterated dopamine , or a pharmaceutically acceptable salt thereof is a compound of Formula I: 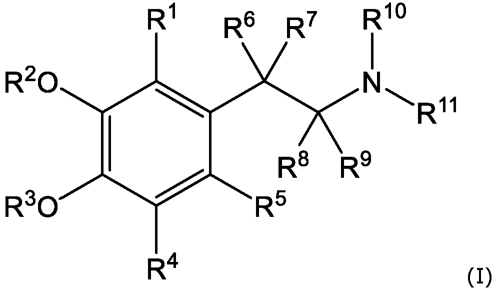
or a pharmaceutically acceptable salt thereof, wherein
R¹, R², R³, R⁴, R⁵, R⁶, R⁷, R⁸, R¹⁰ and R¹¹ are each independently selected from H and D;
R⁹ is selected from H and D; and
wherein at least one of R¹ to R¹¹ is D.

In some embodiments, R⁶ and R⁸ are D. In some embodiments, R⁶, R⁷ and R⁸ are D. In some embodiments, R⁶, R⁷, R⁸ and R⁹ are D.

In some embodiments, R⁹ is H or D; preferably D. In some embodiments, R⁶, R⁷, R⁸ and R⁹ are D. In some embodiments, R¹, R², R³, R⁴, R⁵, R¹⁰ and R¹¹ are H. In preferred embodiments, R⁶, R⁷, R⁸ and R⁹ are D; and R¹, R², R³, R⁴, R⁵, R¹⁰ and R¹¹ are H.

While various levels of deuterium enrichment are contemplated by the invention, positions occupied by D independently have a deuterium enrichment of no less than about 80%, 85%, 90%, 95%, 98% or 100% (and all integers therebetween); especially no less than about 98%. Levels of deuterium enrichment can be determined using conventional analytical methods known to a person of ordinary skill in the art, including mass spectrometry and nuclear magnetic resonance spectroscopy.

Provided is a compound of Formula I whichmay be selected from the group consisting of 2-(3,4-dihydroxyphenyl)ethyl-1,1,2,2,d₄-amine (dopamine-1,1,2,2-d₄); 2-(3,4-dihydroxyphenyl)ethyl-1-deutero-amine; 2-(3,4-dihydroxyphenyl)ethyl-2,2-dideutero-amine; 2-amino-2-deutero-3-(3,4-dihydroxyphenyl) propionic acid; 2-amino-2,3-dideutero-3-(3,4-dihydroxyphenyl) propionic acid; 2-amino-2,3,3-trideutero-3-(3,4-dihydroxyphenyl) propionic acid; 2-amino-3,3-dideutero-3-(3,4-dihydroxyphenyl) propionic acid; 2-amino-3,3-dideutero-3-(3,4-dideuteroxyphenyl) propionic acid; 2-amino-2-deutero-3-(2,3,6-trideutero-4,5-dihydroxyphenyl) propionic acid; 2-amino-2,3-dideutero-3-(2,3,6-trideutero-4,5-dihydroxyphenyl) propionic acid; 2-amino-2,3,3-trideutero-3-(2,3,6-trideutero-4,5-dihydroxyphenyl) propionic acid; 2-amino-2,3,3-trideutero-3-(2,3,6-trideutero-4,5-dideuteroxyphenyl) propionic acid; and pharmaceutically acceptable salts thereof; especially 2-(3,4-dihydroxyphenyl)ethyl-1,1,2,2,d₄-amine; 2-amino-2,3-dideutero-3-(3,4-dihydroxyphenyl) propionic acid; 2-amino-2,3,3-trideutero-3-(3,4-dihydroxyphenyl) propionic acid; and pharmaceutically acceptable salts thereof; most especially 2-(3,4-dihydroxyphenyl)ethyl-1,1,2,2,d₄-amine and a pharmaceutically acceptable salt thereof.

The amount of dopamine, deuterated dopamine, or pharmaceutically acceptable salt thereof in the composition may depend on the visual disorder being treated, the characteristics of the subject such as weight and age, and the route of administration. In some embodiments, the dopamine, deuterated dopamine, or pharmaceutically acceptable salt thereof in the composition is in an amount in the range of from 0.0001% to 60% w/v, 0.001% to 50% w/v, 0.01% to 40% w/v, 0.02% to 30% w/v, 0.03% w/v to 25% w/v, 0.04% to 20% w/v, 0.05% to 15% w/v, 0.06% to 10% w/v, 0.065% to 9% w/v, 0.07% to 8% w/v, 0.075% to 7% w/v, 0.08% to 6% w/v, 0.085% to 5% w/v, 0.09% to 4% w/v, 0.095% to 3% w/v, 0.1% to 2% w/v or 0.105% to 1% w/v of the composition (and all integers therebetween); especially about 0.1%, 0.2%, 0.3%, 0.4%, 0.5%, 0.6%, 0.7%, 0.8%, 0.9% or 1% w/v of the composition.

In preferred embodiments, the dopamine, deuterated dopamine, or pharmaceutically acceptable salt thereof is in solubilized form in the composition. A skilled person will be well aware of procedures routinely used in the art to determine the solubility of a compound, for example, the procedures described in Goodwin (2006) Drug Discovery Today: Technologies, 3(1): 67-71; Jouyban (2010) Handbook of Solubility Data for Pharmaceuticals (CRC Press); or Hefter and Tomkins (2003) The Experimental Determination of Solubilities (John Wiley & Sons, Ltd). For example, the solubility of a compound may be analyzed using UV spectroscopy or high performance liquid chromatography.

In some embodiments, dopamine may be in the form of a pharmaceutically acceptable salt and/or solvate thereof. Also provided is dopamine which may be in the form of a hydrate. In some embodiments, the pharmaceutically acceptable salt of dopamine is the hydrochloride salt, such as that available from Sigma-Aldrich Co. LLC. Additionally provided is a prodrug which may be an ester, and/or an amide prodrug. The prodrug of dopamine may be docarpamine (N-(N-acetyl-L-methionyl)-3,4-bis(ethoxycarbonyl)dopamine, as described in Yoshikawa et al. (1995) Hypertens Res, 18(Suppl 1): S211-S213; a compound described in Haddad et al. (2018) Molecules, 23(1): 40 (doi:10.3390/molecules23010040), such as an ester prodrug e.g. a lipophilic 3,4-O-diester prodrug of dopamine as described in Casagrande and Ferrari (1973) Farmaco Sci, 28(2): 143-148, and Borgman et al. (1973) J Med Chem, 16(6): 630-633; an amide prodrug described in Peura et al. (2013) Pharm Res, 30: 2523-2537, Tutone et al. (2016) Eur J Med Chem, 124: 435-444, or US Patent No. 4,064,235; or a compound described in US Patent No. 4,311,706.

In some embodiments, the deuterated dopamine may be a pharmaceutically acceptable salt and/or solvate thereof. Also provided is deuterated dopamine or pharmaceutically acceptable salt thereof which may be in the form of a hydrate. In some embodiments, the pharmaceutically acceptable salt of deuterated dopamine is the hydrochloride salt, such as dopamine-1,1,2,2-d₄ hydrochloride available from Sigma-Aldrich Co. LLC. Additionally provided are deuterated derivatives of the salts described in US 2007/0027216 A1. Further provided is a prodrug which may be an ester, and/or an amide prodrug. The prodrug may be an ester prodrug of the deuterated compound, such as a deuterated derivative of (2R)-2-phenylcarbonyloxypropyl(2*S*)-2-amino-3-(3,4-dihydroxyphenyl)propanoate mesylate as described in US 2009/0156679 A1, a deuterated derivative of levodopa methyl ester or levodopa ethyl ester as described in US 2014/0088192 A1, a deuterated derivative of XP21279 described in LeWitt et al. (2012) Clin Neuropharmacol, 35: 103-110, and a deuterated derivative of an ester prodrug described in Haddad et al. (2018) Molecules, 23(1): 40 (doi:10.3390/molecules23010040) including a deuterated derivative of lipophilic 3,4-O-diester prodrug of dopamine as described in Casagrande and Ferrari (1973) Farmaco Sci, 28(2): 143-148, and Borgman et al. (1973) J Med Chem, 16(6): 630-633; docarpamine (N-(N-acetyl-L-methionyl)-3,4-bis(ethoxycarbonyl)dopamine, as described in Yoshikawa et al. (1995) Hypertens Res, 18(Suppl 1): S211-S213; or an amide prodrug of the deuterated compound, such as a deuterated derivative of levodopa amide, levodopa carboxamide or levodopa sulfonamide as described in US 2014/0088192 A1, a deuterated derivative of an amide prodrug described in Haddad et al. (2018) Molecules, 23(1): 40 (doi:10.3390/molecules23010040), a deuterated derivative of an amino acid prodrug described in Wang et al. (2013) J Food Drug Anal, 21: 136-141, Zhou et al. (2013) Bioorganic Med Chem Lett, 23: 5279-5282 and Zhou et al. (2010) Eur J Med Chem, 45: 4035-4042, a deuterated derivative of an amide prodrug described in Atlas et al. (2016) CNS Neurosci Ther, 22: 461-467, a deuterated derivative of an amide prodrug described in Peura et al. (2013) Pharm Res, 30: 2523-2537, a deuterated derivative of an amide prodrug described in Tutone et al. (2016) Eur J Med Chem, 124: 435-444, or derivative of an amide prodrug described in US Patent No. 4,064,235; a deuterated derivative of a phosphate prodrug described in WO 2016/065019; or a deuterated derivative of a compound described in US Patent No. 4,311,706.

In some embodiments, the composition further comprises an antioxidant. The antioxidant may be any compound that slows down, inhibits or prevents the oxidation of any component of the composition of the invention, especially dopamine, deuterated dopamine, or pharmaceutically acceptable salt thereof. Suitable antioxidants may include, but are not limited to, ascorbic acid or vitamin C, phenolic acids, sorbic acid, sodium bisulfite, sodium metabisulfite, sodium thiosulfate, acetyl cysteine, ethylene diamine tetraacetic acid (EDTA), sodium nitrite, ascorbyl stearate, ascorbyl palmitate, alpha-thioglycerol, erythorbic acid, cysteine hydrochloride, citric acid, tocopherol or vitamin E, tocopherol acetate, dibutylhydroxytoluene, soybean lecithin, sodium thioglycolate, butylhydroxyanisole, propyl gallate, uric acid, melatonin, thiourea, or salts or combinations thereof. In some embodiments, the antioxidant is ascorbic acid or a salt thereof.

The antioxidant may be present in an amount suitable to substantially slow down, inhibit or prevent oxidation of any component of the composition of the invention, especially dopamine, deuterated dopamine, or pharmaceutically acceptable salt thereof. For example, the antioxidant may be present in an amount in the range of from 0.01% to 10% w/v, 0.01% to 5% w/v, 0.03% to 4% w/v, 0.05% to 3% w/v, 0.07% to 2% w/v, 0.09% to 1% w/v or 0.1% to 0.5% w/v of the composition; especially in an amount of about 0.1% w/v of the composition.

In some embodiments, the composition further comprises a pharmaceutically acceptable carrier. Suitable pharmaceutically acceptable carriers include, but are not limited to, aqueous carriers, oils, fatty acids, a silicone liquid carrier such as a perfluorocarbon or fluorinated liquid carrier, for example, as described in US Patent No. 6,458,376 B1, and combinations thereof.

In some embodiments, the composition of the invention comprises an oil. Suitable oils include, but are not limited to, almond oil; castor oil; mineral oil; olive oil; peanut oil; coconut oil; soybean oil; corn oil; anise oil; clove oil; cassia oil; cinnamon oil; arachis oil; maize oil; caraway oil; rosemary oil; peppermint oil; eucalyptus oil; seed oils such as canola oil, cottonseed oil, linseed oil, safflower oil, sesame oil or sunflower oil; silicone oil; or combinations thereof. In some embodiments, the oil may be included in the composition in the form of an oil-in-water emulsion, optionally with a surfactant, and an aqueous carrier. The oil may be present in an amount in the range of from about 0.1% to 20% w/v of the composition.

In some embodiments, the carrier is an aqueous carrier. The aqueous carrier is preferably a pharmaceutically acceptable aqueous carrier. A variety of pharmaceutically acceptable aqueous carriers well known in the art may be used. For example, the aqueous carrier may be selected from, but is not limited to, saline, water, aqueous buffer, an aqueous solution comprising water and a miscible solvent, and combinations thereof. In some embodiments, the aqueous carrier is saline. When saline is used, it is preferably isotonic for the point of administration, such as the eye. For example, in some embodiments the saline comprises 0.15 to 8% w/v sodium chloride; especially 0.18% to 7% w/v, 0.22% to 5% w/v or 0.45% to 3% w/v sodium chloride; more especially 0.5 to 2% w/v or 0.65% to 1.5% w/v sodium chloride; most especially about 0.9% w/v sodium chloride.

In some embodiments where the aqueous carrier is not isotonic, for example water, the composition may contain a tonicity agent. Any pharmaceutically acceptable tonicity agent well known in the art may be used. Suitable tonicity agents include, but are not limited to, boric acid, sodium acid phosphate buffer, sodium chloride, glucose, trehalose, potassium chloride, calcium chloride, magnesium chloride, polypropylene glycol, glycerol, mannitol, or salts or combinations thereof. The tonicity agent may be present in the composition in an amount that provides isotonicity with the point of administration, such as the eye, for example in the range of from 0.02 to 15% w/v.

In some embodiments the carrier is a buffer, wherein the buffer maintains a pH in the range of from 4 to 8, 5 to 7, 5.5 to 6.5, or about 5.5, 6.0 or 6.5. Suitable buffering agents include, but are not limited to, acetic acid, citric acid, sodium metabisulfite, histidine, sodium bicarbonate, sodium hydroxide, boric acid, borax, alkali metal phosphates, phosphate or citrate buffers, or combinations thereof. The buffering agent may be present in the composition in an amount suitable to maintain the desired pH.

In some embodiments, the pH of the composition is in the range of from 4 to 8, 5 to 7, 5.5 to 6.5, or about 5.5, 6.0 or 6.5.

In some embodiments, the composition may further comprise an inhibitor of aromatic L-amino acid decarboxylase. Suitable inhibitors of aromatic L-amino acid decarboxylase include, but are not limited to, carbidopa, benserazide, methyldopa, or salts or combinations thereof. In some embodiments, the inhibitor of aromatic L-amino acid decarboxylase is carbidopa.

The amount of the inhibitor of aromatic L-amino acid decarboxylase in the composition of the invention will depend on the condition being treated, the route of administration of the composition and the amount of deuterated levodopa in the composition. The inhibitor of aromatic L-amino acid decarboxylase should be present in an amount sufficient to substantially inhibit the decarboxylation of levodopa or a pharmaceutically acceptable salt thereof. In some embodiments, the ratio of levodopa or pharmaceutically acceptable salt thereof to the inhibitor of aromatic L-amino acid decarboxylase may be in the range of from 20:1 to 1:1, 15:1 to 1:1, 10:1 to 1:1, 9:1 to 1:1, 8:1 to 1:1, 7:1 to 1:1, 6:1 to 2:1 or 5:1 to 3:1. In some embodiments, the ratio of levodopa or a pharmaceutically acceptable salt thereof to the inhibitor of aromatic L-amino acid decarboxylase is about 4:1.

In some embodiments, the inhibitor of aromatic L-amino acid decarboxylase in the composition is in an amount in the range of from 0.0005% to 30% w/v, 0.0025% to 15% w/v, 0.005% to 12.5% w/v, 0.0075% to 10% w/v, 0.01% to 7.5% w/v, 0.0125% to 5% w/v, 0.015% to 2.5% w/v, 0.0163% to 2.25% w/v, 0.0175% to 2% w/v, 0.0188% to 1.75% w/v, 0.02% to 1.5% w/v, 0.0213% to 1.25% w/v, 0.0225% to 1% w/v, 0.0238% to 0.75% w/v, 0.025% to 0.5% w/v, 0.0263% to 0.25% w/v of the composition (and all integers therebetween); especially about 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.125%, 0.15%, 0.175%, 0.2%, 0.225% or 0.25% w/v of the composition.

The composition may also comprise or may be administered separately, simultaneously or sequentially with one or more ancillary pharmaceutically active agents. In some embodiments, the ancillary pharmaceutically active agent may increase activation of the dopaminergic system. Exemplary ancillary pharmaceutically active agents include, but are not limited to, a dopamine receptor agonist, a gamma-aminobutyric acid (GABA) receptor antagonist and/or a muscarinic acetylcholine receptor antagonist. In some embodiments, the pharmaceutically active agent is an agent that is used for inhibiting the development or progression of a visual disorder, particularly a visual disorder involving reduced dopamine levels in the eye, such as myopia.

In some embodiments, the composition of the invention further comprises a dopamine receptor agonist. The dopamine receptor agonist may have agonist activity at any dopamine receptor subtype, including, but not limited to, any receptor subtype from the D₁-like (D₁ and D₅ receptors) and D₂-like (D₂, D₃ and D₄ receptors) families of receptors, and dopamine receptor heterodimers. Suitable dopamine receptor agonists include, but are not limited to, quinpirole, apomorphine, ropinirole, pramipexole, dexpramipexole, piribedil, rotigotine, bromocriptine, lisuride, cabergoline, 2-amino-6,7-dihydroxy-1,2,3,4-tetrahydronaphthalene (ADTN), pergolide, calidopa, dihydrexidine, doxathrine, propylnorapomorphine, quinagolide, roxindole, sumanirole, fenoldopam, ergocornine, 1-phenyl-2,3,4,5-tetrahydro-(1H)-3-benzazepine-7,8-diol (also known as SKF-38393), 2-(N-phenethyl-N-propyl)amino-5-hydroxytetralin (PPHT; also known as N-0434), dihydroergotamine, (1*R,*3*S*)-1-(aminomethyl)-3-phenyl-3,4-dihydro-1H-isochromene-5,6-diol (also known as A-68930), carmoxirole, fenoldopam, or salts or combinations thereof. In some embodiments, the dopamine receptor agonist is dihydroergotamine tartrate, 2-(N-phenethyl-N-propyl)amino-5-hydroxytetralin hydrochloride or (1*R*,3*S*)-1-(aminomethyl)-3-phenyl-3,4-dihydro-1*H*-isochromene-5,6-diol hydrochloride. In some embodiments, the dopamine receptor agonist is selected from ADTN, quinpirole, apomorphine, and salts and combinations thereof; especially ADTN and salts thereof. In some embodiments, the composition further comprises dopamine, levodopa or a pharmaceutically acceptable salt thereof.

The amount of dopamine receptor agonist in the composition may depend on the condition being treated and the route of administration. In some embodiments, the dopamine receptor agonist in the composition is in an amount in the range of from 0.01% to 20% w/v, 0.01% to 10% w/v, 0.01% to 5% w/v, 0.03% to 3% w/v, 0.033% to 2.7% w/v, 0.038% to 2.4% w/v, 0.043% to 2.1% w/v, 0.05% to 1.8% w/v, 0.06% to 1.5% w/v, 0.075% to 1.2% w/v, 0.1% to 0.9% w/v or 0.15 to 0.6% w/v of the composition (and all integers therebetween); especially about 0.2%, 0.21%, 0.22%, 0.23%, 0.24%, 0.25%, 0.26%, 0.27%, 0.28%, 0.29%, 0.3%, 0.31%, 0.32%, 0.33%, 0.34%, 0.35%, 0.36%, 0.37%, 0.38%, 0.39%, or 0.4% w/v of the composition.

In some embodiments, the composition of the invention further comprises a GABA receptor antagonist. The GABA receptor antagonist may have antagonist activity at any GABA receptor subtype, including, but not limited to, GABA_{A}, GABA_{B} and/or GABA_{A-}rho (formerly GABA_{C}) receptors. Suitable GABA receptor antagonists include, but are not limited to, bicuculline, flumazenil, gabazine, phenylenetetrazol, (1,2,5,6-tetrahydropyridin-4-yl)methylphosphinic acid (TPMPA), (3-aminopropyl)(cyclohexylmethyl)phosphinic acid (also known as CGP-46381), 4-imidazoleacetic acid, picrotoxin, piperidin-4-ylphosphinic acid (PPA), piperidin-4-ylseleninic acid (SEPI), 3-aminopropyl-*N*-butylphosphinic acid (also known as CGP-36742), (piperidin-4-yl)methylphosphinic acid (P4MPA), or salts or combinations thereof. In some embodiments, the GABA receptor antagonist is selected from TPMPA, bicuculline and salts and combinations thereof; especially TPMPA and salts thereof.

The amount of GABA receptor antagonist in the composition may depend on the condition being treated and the route of administration. In some embodiments, the GABA receptor antagonist in the composition is in an amount in the range of from 0.01% to 20% w/v, 0.01% to 10% w/v, 0.01% to 5% w/v, 0.03% to 3% w/v, 0.033% to 2.7% w/v, 0.038% to 2.4% w/v, 0.043% to 2.1% w/v, 0.05% to 1.8% w/v, 0.06% to 1.5% w/v, 0.075% to 1.2% w/v, 0.1% to 0.9% w/v or 0.15 to 0.6% w/v of the composition (and all integers therebetween); especially about 0.2%, 0.21%, 0.22%, 0.23%, 0.24%, 0.25%, 0.26%, 0.27%, 0.28%, 0.29%, 0.3%, 0.31%, 0.32%, 0.33%, 0.34%, 0.35%, 0.36%, 0.37%, 0.38%, 0.39%, or 0.4% w/v of the composition.

In some embodiments, the composition of the invention further comprises a muscarinic acetylcholine receptor antagonist. The muscarinic acetylcholine receptor antagonist may have antagonist activity at any muscarinic acetylcholine receptor subtype, including, but not limited to, M₁, M₂, M₃, M₄ and M₅ receptors. Suitable muscarinic receptor antagonists include, but are not limited to, atropine, pirenzepine, himbacine, hyoscine, cyclopentolate, ipratropium, oxitropium, tropicamide, oxybutynin, tolterodine, diphenhydramine, dicycloverine, flavoxate, tiotropium, trihexyphenidyl, solifenacin, darifenacin, benzatropine, mebeverine, procyclidine, aclidinium, muscarinic toxin 1 (MT1), muscarinic toxin 2 (MT2), muscarinic toxin 3 (MT3), muscarinic toxin 4 (MT4), muscarinic toxin 7 (MT7), or salts or combinations thereof. In some embodiments, the muscarinic acetylcholine receptor antagonist is selected from atropine, pirenzepine, himbacine, and salts and combinations thereof; especially atropine and pirenzepine and salts and combinations thereof.

The amount of muscarinic acetylcholine receptor antagonist in the composition may depend on the condition being treated and the route of administration. In some embodiments, the muscarinic acetylcholine receptor antagonist in the composition is in an amount in the range of from 0.0001% to 30% w/v, 0.0003% to 25% w/v, 0.0005% to 20% w/v, 0.0007% to 15% w/v, 0.0009% to 10% w/v, 0.001% to 5% w/v, 0.003% to 1%, 0.005% to 0.5%, 0.007% to 0.2% w/v, or 0.009% to 0.1% of the composition (and all integers therebetween); especially about 0.009%, 0.01%, 0.02%, 0.03%, 0.04%, 0.05%, 0.06%, 0.07%, 0.08%, 0.09% or 0.1% w/v of the composition.

The composition of the invention may further comprise a surfactant. A variety of pharmaceutically acceptable surfactants well known in the art may be used. Exemplary surfactants include, but are not limited to, surfactants of the following classes: alcohols; amine oxides; block polymers; carboxylated alcohol or alkylphenol ethoxylates; carboxylic acids/fatty acids; ethoxylated arylphenols; ethoxylated fatty esters, oils, fatty amines or fatty alcohols such as cetyl alcohol; fatty esters; fatty acid methyl ester ethoxylates; glycerol esters such as glycerol monostearate; glycol esters; lanolin-based derivatives; lecithin or derivatives thereof; lignin or derivatives thereof; methyl esters; monoglycerides or derivatives thereof; polyethylene glycols; polypropylene glycols; alkylphenol polyethylene glycols; alkyl mercaptan polyethylene glycols; polypropylene glycol ethoxylates; polyethylene glycol ethers such as Cetomacrogol 1000; polymeric surfactants; propoxylated and/or ethoxylated fatty acids, alcohols or alkylphenols; protein-based surfactants; sarcosine derivatives; sorbitan derivatives such as polysorbates; sorbitol esters; esters of sorbitol polyglycol ethers; fatty acid alkylolamides; *N-*alkylpolyhydroxy fatty acid amide; *N*-alkoxypolyhydroxy fatty acid amide; alkyl polyglycosides; quaternary ammonium compounds such as benzalkonium chloride; cyclodextrins such as alpha-, beta- or gamma-cyclodextrin; sucrose or glucose esters or derivatives thereof; sulfosuccinates such as dioctyl sodium sulfosuccinate; or combinations thereof. Without wishing to be bound by theory, the presence of a surfactant may be useful in emulsifying an aqueous carrier with an oil if an aqueous carrier and oil are included in the composition and may enhance the penetration of the active ingredients, such as dopamine, deuterated dopamine or a pharmaceutically acceptable salt thereof, through the corneal epithelium. The surfactant may be present in an amount in the range of from about 0.1% to 30% w/v of the composition.

In some embodiments, the composition of the invention further comprises a rheology modifier. The rheology modifier may be used to alter the surface tension and flow of the composition and may also contribute to the composition's residence time on the surface of the eye when topically applied. Suitable rheology modifiers are well known in the art. For example, the rheology modifier may be selected from, but is not limited to, hyaluronic acid, chitosan, polyvinyl alcohol, polyethylene glycol, polyvinyl pyrrolidone, dextran, methylcellulose, hydroxypropylmethylcellulose, hydroxyethylcellulose, hydroxypropylcellulose, hydroxypropyl guar, acrylates such as Carbopol polymers, poloxamers, gum arabic, xanthan gum, guar gum, locust bean gum, carboxymethylcellulose, alginate, starch (from rice, corn, potato or wheat), carrageenan, konjac, aloe vera gel, agarose, pectin, tragacanth, curdlan gum, gellan gum, scleroglucan, and derivatives and combinations thereof. The rheology modifier should be present in an amount sufficient to obtain the desired viscosity of the composition. The rheology modifier may be present in an amount in the range of from about 0.5% to 5% w/v of the composition.

The composition of the invention may further comprise a preservative. The preservative may be particularly useful for preventing microbial contamination in a composition which is subject to multiple uses from the same container, for example, if the composition of the invention is formulated for topical administration in a multiple unit dose form. Suitable preservatives include any pharmaceutically acceptable preservative routinely used in the art to prevent microbial contamination in a composition. Non-limiting examples include sodium perborate, stabilized oxychloro complex, polyquaternium-1, phenylmercuric acid, benzalkonium chloride, chlorbutanol, phenylmercuric acetate, phenylmercuric nitrate, chlorhexidine, benzododecinium bromide, cetrimonium chloride, thiomersal, methyl parahydroxybenzoate, propyl parahydroxybenzoate, polyquaternium ammonium chloride, polyaminopropyl biguanide, hydrogen peroxide, benzoic acid, phenolic acids, sorbic acid, benzyl alcohol or salts or combinations thereof. The preservative should be present in an amount that provides adequate preservative activity. For example, the preservative may be present in an amount in the range of from about 0.001% to 1% w/v of the composition.

It may be desirable to increase the permeation of the composition into the eye. Accordingly, in some embodiments, the composition of the invention may further comprise a permeation enhancing agent. Suitable permeation enhancing agents include, but are not limited to, dimethyl sulfoxide (DMSO); cyclodextrins such as alpha-, beta- or gamma-cyclodextrin; EDTA; decamethonium; glycocholate; cholate; saponins; fusidate; taurocholates; polyethylene glycol ethers; polysorbates; or salts, derivatives or combinations thereof. In some embodiments, the permeation enhancing agent is dimethyl sulfoxide. Other permeation enhancing agents include nanoparticles, microemulsions, liposomes or micelles which, in some embodiments, encapsulate one or more components of the composition, including dopamine, deuterated dopamine, or a pharmaceutically acceptable salt thereof. The permeation enhancing agent should be present in an amount that facilitates permeation of dopamine, deuterated dopamine, or a pharmaceutically acceptable salt thereof across the corneal epithelium. For example, the permeation enhancing agent may be present in an amount in the range of from about 0.1% to 30% w/v of the composition.

In particular embodiments, the permeation enhancing agent is a micelle. Suitable micelles include, but are not limited to, a Triton X-100 micelle e.g. the micelle described in Jodko-Piorecka and Litwinienko (2015) Free Radical Biology and Medicine, 83: 1-11; a surfactant nanomicelle e.g. a nanomicelle formed with sodium dodecyl sulfate, dodecyltrimethylammonium bromide, cetyltrimethylammonium bromide, n-dodecyl tetra (ethylene oxide), Vitamin E TGPS, octoxynol-40 and/or dioctanoyl phosphatidylcholine; a polymeric micelle e.g. a micelle formed with poly(caprolactone), poly (D,L-lactide), polypropylene oxide, poly(β-benzyl-1-aspartate), methoxy poly(ethylene glycol)-hexylsubstituted poly(lactide), Pluronic F127 poly(oxyethylene)/poly(oxypropylene)/poly(oxyethylene), Pluronic F 68, Pluronic F 127, poly(hydroxyethylaspartamide)-polyethylene glycol-hexadecylamine, polyoxyl 40 stearate, N-isopropylacrylamide with vinyl pyrrolidone and acrylic acid crosslinked with N,N'-methylene bis-acrylamide, Pluronic F127 and chitosan, poly(lactic acid), poly(glycolic acid), poly(ethylene glycol), poly(ethylene oxide), *N*-phthaloylcarboxymethylchitosan, poly(2-ethylhexyl acrylate)-b-poly(acrylic acid), poly(tert-butyl acrylate)-b-poly(2-vinylpyridine), poly(ethylene oxide)-b-polycaprolactone, poly(ε-caprolactone)-b-poly(ethylene glycol)-b-poly(ε-caprolactone), poly(ε-caprolactone)-b-poly(methacrylic acid), poly(ethyleneglycol)-b-poly(ε-caprolactone-co-trimethylenecarbonate), poly(aspartic acid)-b-polylactide, poly(ethylene glycol)-block-poly(aspartate-hydrazide), poly(*N*-isopropylacrylamide-co-methacrylic acid)-g-poly(D,L-lactide) and/or stearic acid-grafted chitosan oligosaccharide; the micelles described in Khuphe et al. (2015) Chem. Commun., 51: 1520-1523; the micelles described in WO 2005/076998 A2; or the micelles disclosed in US 2009/0092665 A1. In particular embodiments, the micelle encapsulates the dopamine, deuterated dopamine, or a pharmaceutically acceptable salt thereof in the composition. In some embodiments, the micelle comprises dopamine, deuterated dopamine, or a pharmaceutically acceptable salt thereof, such as poly{(styrene-alt-maleic acid)-*co*-[styrene-alt-(*N*-3,4-dihydroxyphenylethyl-maleamic acid)]} as described in Chenglin et al. (2012) Langmuir, 28: 9211-9222,.

In some embodiments, the permeation enhancing agent is a liposome. Suitable liposomes include, but are not limited to, a liposome prepared from dipalmitoyl phosphatidylcholine, such as egg phosphatidylcholine; and the liposomes described in Zhigaltsev et al. (2001) J Liposome Res, 11(1): 55-71; Jain et al. (1998) Drug Dev Ind Pharm, 24(7): 671-675; WO 2014/076709 A1; Chonn et al. (1995) Curr Opin Biotechnol, 6: 698-708; Lasic (1998) Trends Biotechnol, 16: 307-321; Gregoriadis (1995) Trends Biotechnol, 13: 527-537; Szoka and Papahadjopoulos (1980) Ann Rev Biophys Bioeng, 9: 467-508; US Patent No. 4,235,871; US Patent No. 4,837,028; and US Patent Publication No. 2004/0224010 A1.

The composition of the invention may also further comprise a chelating agent. Suitable chelating agents include, but are not limited to, amino carboxylic acids or salts thereof such as EDTA, nitrilotriacetic acid, nitrilotripropionic acid, diethylenetriamine pentacetic acid, 2-hydroxyethyl-ethylenediamine-triacetic acid, 1,6-diamino-hexamethylene-tetraacetic acid, 1,2-diamino-cyclohexane tetraacetic acid, *O,O*'-bis(2-aminoethyl)-ethyleneglycol-tetraacetic acid, 1,3-diaminopropane-tetraacetic acid, *N,N-*bis(2-hydroxybenzyl)ethylenediamine-*N,N*-diacetic acid, ethylenediamine-*N,N'*-diacetic acid, ethylenediamine-*N,N*'-dipropionic acid, triethylenetetraamine hexaacetic acid, 7,19,30-trioxa-1,4,10,13,16,22,27,33-octaazabicyclo[11,11,11]pentatriacontane (O-bis-tren), ethylenediamine-*N,N*'-bis(methylenephosphonic acid), iminodiacetic acid, *N,N*-bis(2-hydroxyethyl)glycine (DHEG), 1,3-diamino-2-hydroxypropane-tetraacetic acid, 1,2-diaminopropane-tetraacetic acid, ethylenediamine-tetrakis(methylenephosphonic acid), *N*-(2-hydroxyethyl)iminodiacetic acid, or combinations or salts thereof; especially pharmaceutically acceptable salts or mixed salts of EDTA, such as disodium, trisodium, tetrasodium, dipotassium, tripotassium, lithium, dilithium, ammonium, diammonium, calcium or calcium-disodium; most especially disodium EDTA. The chelating agent may be present in an amount in the range of from about 0.01% to 1% w/v of the composition.

The composition of the invention may further comprise any other pharmaceutically acceptable excipient commonly present in topical or injectable ocular formulations. For example, the compositions may further comprise an alcohol such as isopropanol, benzyl alcohol, cetearyl alcohol or ethanol; a lubricant such as glucose, glycerol, polyethylene glycol, polypropylene glycol or derivatives thereof; a polysaccharide such as chitosan, chitin, dermatan, hyaluronate, heparin, chondroitin, cyclodextrin or derivatives thereof; or combinations thereof.

In some embodiments, the composition of the invention is formulated for topical administration to the eye. In this regard, the composition of the invention may be in the form of an eye drop or gel; especially an eye drop. Without wishing to be bound by theory, formulating the composition for topical administration to the eye is thought to increase user compliance, particularly when the composition is used as a preventative or control measure. This may be particularly important if the composition is administered to a child subject. Furthermore, such a formulation may reduce the incidence of off target effects of dopamine, deuterated dopamine, or a pharmaceutically acceptable salt thereof.

In some embodiments, the composition of the invention is formulated for penetration of dopamine, deuterated dopamine, or a pharmaceutically acceptable salt thereof through the corneal epithelium. In preferred embodiments, greater than about 10%, 20%, 30%, 40%, 50%, 60%, 70% or 80% of the dose of dopamine, deuterated dopamine, or a pharmaceutically acceptable salt thereof penetrates the corneal epithelium.

When formulated as an eye drop or gel, the composition of the invention may be in a single unit dose or multiple unit dose form, preferably a multiple unit dose form.

Also provided is a composition which may be formulated for direct injection into the eye. For example, the composition may be formulated for intravitreal, subconjunctival, intracameral, intrascleral, intracorneal or subretinal injection; especially intravitreal, intrascleral or intracorneal injection. For example, the composition may be formulated for suprachoroidal injection or the composition may also be formulated for injection via a microneedle, for example, via intrascleral or intracorneal administration.

Other excipients and components of the composition may be readily determined by a person skilled in the art. Techniques for formulation and administration may be found in, for example, Remington (1980) Remington's Pharmaceutical Sciences, Mack Publishing Co., Easton, Pa., latest edition; and suitable excipients may be found in, for example, Katdare and Chaubel (2006) Excipient Development for Pharmaceutical, Biotechnology and Drug Delivery Systems (CRC Press).

A person skilled in the art would be familiar with the components of the compositions of the invention and, accordingly, would readily be able to synthesize or source the components, such as from, for example, Sigma Aldrich Co. LLC. For example, dopamine in the form of dopamine hydrochloride is commercially available from a number of sources, such as Sigma-Aldrich Co. LLC, and a synthetic route is available in, for example, Carter et al. (1982) Analytical Profiles of Drug Substances, 11: 257-272.

Deuterated dopamine in the form of dopamine-1,1,2,2-d₄ hydrochloride is commercially available from Sigma-Aldrich Co. LLC, and a synthetic route for deuterated dopamine is available in, for example, Binns et al. (1970) J Chem Soc (C), 8: 1134-1138; WO 2004/056724 A1; WO 2007/093450 A1; WO 2014/122184 A1. Deuterium can be introduced into a compound using synthetic techniques that employ deuterated reagents and/or by exchange techniques, both of which are routine techniques in the art.

The compositions of the invention may be prepared by mixing the components, for example, in a pharmaceutically acceptable carrier or diluent, and adjusting the pH of the composition to a pH in the range of from 4 to 8, 5 to 7, 5.5 to 6.5, or about 5.5, 6.0 or 6.5, if required. The pH of the compositions may be adjusted using any pharmaceutically acceptable pH adjusting agent that is routinely used in the art, such as hydrochloric acid, sodium hydroxide, etc. A person skilled in the art will be well aware of suitable agents.

The composition of the invention may also be sterilized prior to use, for example, by filtration, autoclaving and/or gamma irradiation.

### 4. Methods for Preventing and Treating a Visual Disorder

The compositions of the invention are useful for inhibiting the progression or development of a visual disorder in a subject, particularly a visual disorder involving reduced dopamine levels in the eye, such as a visual disorder associated with diabetic retinopathy or Parkinson's disease, or myopia. Accordingly, the compositions of the invention may be used in methods of inhibiting the progression or development of a visual disorder in a subject. The compositions of the invention may also be used in the manufacture of a medicament for the uses described herein.

The compositions of the invention are useful for inhibiting the progression of a visual disorder in a subject. In this regard, the compositions of the invention may be used for treating a visual disorder. In some embodiments, the compositions of the invention may slow the progression of a visual disorder in a subject.

The compositions of the invention are also useful for inhibiting the development of a visual disorder in a subject. Thus, the compositions of the invention are useful for preventing a visual disorder in a subject. In some embodiments, the compositions of the invention may delay the onset of a visual disorder in a subject, i.e. may increase the age of the subject at which the visual disorder is developed and, therefore, the possible severity of the visual disorder.

The visual disorder may be any visual disorder involving reduced dopamine levels in the eye, particularly reduced dopamine levels in the retina. Accordingly, the visual disorder may be any visual disorder where increasing dopamine levels in the eye, particularly the retina, is associated with effective inhibition of the progression or development of the visual disorder.

There are numerous visual disorders involving reduced dopamine levels in the eye. For example, the visual disorder may be, but is not limited to, a visual disorder associated with diabetic retinopathy or Parkinson's disease, myopia, increased ocular growth, reduced spatial and temporal contrast sensitivity, amblyopia, blurred or double vision, eye strain, trouble with voluntarily opening the eyes (apraxia), eyelid spasms (blepharospasm), excessive blinking, altered color perception, reduced depth perception or visual hallucinations. In some embodiments, the visual disorder is selected from a visual disorder associated with diabetic retinopathy or Parkinson's disease, and myopia. In particular embodiments, the visual disorder is myopia.

Also provided are visual disorders which may be selected from the group consisting of a visual disorder associated with diabetic retinopathy or Parkinson's disease, myopia, increased ocular growth, reduced spatial and temporal contrast sensitivity, amblyopia, blurred or double vision, eye strain, trouble with voluntarily opening the eyes (apraxia), eyelid spasms (blepharospasm), excessive blinking, altered color perception, reduced depth perception, retinitis pigmentosa, age-related macular degeneration, or visual hallucinations. In some embodiments, the visual disorder is selected from a visual disorder associated with diabetic retinopathy or Parkinson's disease, retinitis pigmentosa, age-related macular degeneration and myopia. In particular embodiments, the visual disorder is myopia.

In some embodiments, the visual disorder is not associated with Parkinson's disease.

In particular embodiments, the visual disorder is a disorder of the posterior segment of the eye. Suitable disorders include, but are not limited to, a visual disorder associated with diabetic retinopathy or Parkinson's disease, retinitis pigmentosa, age-related macular degeneration and myopia.

A visual disorder associated with Parkinson's disease includes, but is not limited to, reduced visual acuity, reduced contrast sensitivity, and/or disordered color discrimination.

A visual disorder associated with diabetic retinopathy includes, but is not limited to, reduced visual acuity, reduced contrast sensitivity and reduced peripheral visual field.

The method includes administering the composition of the invention to a subject. The composition of the invention is to be administered locally through topical administration to the surface of the eye

In some embodiments, the composition is topically administered to the eye, for example, in the form of an eye drop or gel. In preferred embodiments, the composition is applied as an eye drop. The composition of the invention may be applied to any surface of the eye, preferably the cornea/sclera, thereby allowing the components present in the composition, particularly dopamine, deuterated dopamine, or a pharmaceutically acceptable salt thereof, to penetrate into the eye. In some embodiments, the composition is formulated such that dopamine, deuterated dopamine, or a pharmaceutically acceptable salt thereof penetrates through the corneal epithelium.

Also provided is a composition that is administered by injection into the eye. For example, the composition may be injected directly into the sclera, anterior chamber or vitreous, or may be injected into the subconjunctival, peribulbar, retrobulbar or suprachoroidal space. For example, the composition may be administered via intravitreal, subconjunctival, intracameral, intrascleral, intracorneal or subretinal injection; especially intravitreal, intrascleral or intracorneal injection. , The composition may be administered via suprachoroidal injection. The composition may also be administered by intravitreal injection. The composition may be injected using a microneedle, for example, via intrascleral or intracorneal administration. For administration via these routes, the composition may be in the form of a sterile injectable solution.

The portion of the eye onto which the composition of the invention is preferably administered is the portion that allows for penetration of the components, particularly dopamine, deuterated dopamine, or a pharmaceutically acceptable salt thereof, into the eye, preferably into the retina. Administration is preferably performed on the cornea/sclera and conjunctiva for topical administration. Also provided is a composition which may be injected into the subconjunctival, peribulbar, retrobulbar or suprachoroidal space, or into the sclera, cornea, anterior chamber or vitreous.

The composition of the invention may be used with both hard and soft contact lenses.

Dosage regimes may be established for different indications in accordance with methodologies well known to a person skilled in the art. The dosage of the composition will depend on the condition to be treated, the age of the subject and the route of administration.

The composition of the invention may be administered topically in a suitable amount so as to provide a dose of dopamine, deuterated dopamine, a pharmaceutically acceptable salt thereof in the range of from 0.001 mg/kg/day to 12 mg/kg/day, especially from 0.001 mg/kg/day to 4 mg/kg/day, more especially from 0.001 mg/kg/day to 2 mg/kg/day. In some embodiments the composition is administered in a suitable amount so as to provide a dose of dopamine, deuterated dopamine, or a pharmaceutically acceptable salt thereof in the range of from 0.001 mg/kg/day to 30 mg/kg/day, especially from 0.001 mg/kg/day to 12 mg/kg/day, more especially from 0.001 mg/kg/day to 4 mg/kg/day, most especially from 0.001 mg/kg/day to 2 mg/kg/day.

When administered topically as an eye drop, the composition of the invention may be administered in an amount in the range of from 1 to 6 drops per eye (and all integers therebetween), which may equate to, for example, an amount in the range of from about 0.04 mL to 0.24 mL per eye (and all integers therebetween). Drops may be applied to each eye from 1 to 4 times daily. When the composition of the invention is formulated as a gel, an equivalent dose is provided. A skilled person will be aware of suitable dispensers for topical application of the composition of the invention.

When administered by injection, a provided composition may be administered in an amount in the range of from 0.001 mL to 0.5 mL (and all integers therebetween), especially about 0.01 mL. The composition of the invention may be administered at a frequency of once per week to once daily.

In order that the invention may be readily understood and put into practical effect, particular preferred embodiments will now be described by way of the following non-limiting examples.

### EXAMPLES

### EXAMPLE 1 - PREPARATION OF DOPAMINE COMPOSITIONS

To make a 150 mM stock solution, 28.4 mg dopamine (in the form of dopamine hydrochloride, commercially available from Sigma-Aldrich Co. LLC) was dissolved in 1 mL of a solution containing 0.1% ascorbic acid in 1x PBS (pH approximately 5.5). The stock solution was further diluted in an appropriate volume of a solution containing 0.1% ascorbic acid in 1x PBS to generate 0.15 mM (0.0028% w/v), 1.5 mM (0.028% w/v) and 15 mM (0.28% w/v) solutions.

Combination solutions were prepared by adding the appropriate amount of atropine (in the form of atropine sulfate monohydrate, commercially available from Sigma-Aldrich Co. LLC), pirenzepine (in the form of pirenzepine dihydrochloride, commercially available from Sigma-Aldrich Co. LLC) or TPMPA ((1,2,5,6-tetrahydropyridin-4-yl)methyl phosphinic acid in the form of TPMPA hydrate, commercially available from Sigma-Aldrich Co. LLC) to a 1 mL solution of dopamine prepared above.

### EXAMPLE 2 - EFFECT OF DOPAMINE COMPOSITIONS ON FORM DEPRIVATION MYOPIA DEVELOPMENT

30 white male cockerel chickens were randomly assigned to one of six treatment groups as defined below (n = 5 per group) and were treated for a four day period.
- Chicks fitted with a translucent diffuser over their left eye to induce form-deprivation myopia (FDM);
- Chicks fitted with a translucent diffuser over their left eye and daily intravitreal injection of a 1.5 mM (0.028%) dopamine solution prepared according to Example 1;
- Chicks fitted with a translucent diffuser over their left eye and twice daily topical administration of a 1.5 mM (0.028%) dopamine solution prepared according to Example 1;
- Chicks fitted with a translucent diffuser over their left eye and daily intravitreal injection of a 15 mM (0.28%) dopamine solution prepared according to Example 1;
- Chicks fitted with a translucent diffuser over their left eye and twice daily topical administration of a 15 mM (0.28%) dopamine solution prepared according to Example 1;
- Age-matched untreated control group.

For the drug treatments, the compositions were administered under light isoflurane anesthesia using intravitreal injection or topical administration.

Intravitreal injection was performed as follows: Using a 30 gauge needle attached to a Hamilton syringe, 10 µL (0.01 mL) of the test composition was injected into the vitreous chamber of the eye once daily.

Topical administration was performed as follows: Two drops of 40 µL (two drops of 0.04 mL, or 0.08 mL total) of the test composition was applied to the corneal surface of the eye using an eye drop dispenser. Drops were applied to the chicks twice daily.

To determine changes in the rate of eye growth and the development of myopia, changes in axial length was assessed. Myopia is associated with excessive elongation of the eye in the axial direction relative to normal growth rates. Axial length, anterior chamber depth and vitreal chamber depth were measured using A-scan ultrasonography (Biometer AL-100; Tomey Corporation, Nagoya, Japan). Statistical analysis of changes in axial length, anterior chamber depth and vitreal chamber depth between groups involved a one-way ANOVA test followed by a Student's T-test with Bonferroni correction. All data are presented as the mean ± the standard deviation of the mean (SEM).

### Results

The results are presented in Figure 1. Administration of the dopamine solution via intravitreal injection significantly inhibited the axial elongation associated with form-deprivation myopia (FDM; 9.12±0.05 mm) (ANOVA, F(3,23)=6.934, p=0.002; Figure 1). Both investigated concentrations of dopamine (1.5 mM and 15 mM) significantly inhibited the axial elongation associated with form-deprivation myopia relative to that seen in diffuser-only treated counterparts (1.5 mM: 8.82±0.02 mm, 78% protection, p<0.05; 15 mM: 8.82±0.11 mm, 78% protection, p<0.05). Furthermore, the axial length of eyes treated with both concentrations of dopamine was not statistically different to eyes of age-matched untreated chickens (8.74±0.04 mm, p=1.000 for both concentrations). Across all conditions there was no significant difference in both anterior chamber depth (ANOVA, F(3,23)=0.348, p=0.791) or lens thickness (ANOVA, F(3,23)=2.613, p=0.077). Instead, alterations in axial length, associated with diffuser-wear and intravitreal injections of dopamine, represented changes in vitreal chamber depth (ANOVA, F(3,23)=6.112, p=0.003).

When the dopamine solution was administered as twice daily topical eye drops, the excessive growth associated with diffuser wear was inhibited (ANOVA, F(3,23)=14.978, p<0.000; Figure 1). Specifically, the excessive growth associated with form-deprivation myopia was inhibited in a dose-dependent manner, with a significant effect observed at a concentration of 15 mM (8.84±0.07 mm, 72% protection relative to FDM-only, p<0.01), at which point diffuser-treated eyes were not statistically different in axial length from untreated control eyes (p=0.191). Across all conditions tested, there was no significant difference in both anterior chamber depth (ANOVA, F(3,23)=0.348, p=0.791) and lens thickness (ANOVA, F(3,23)=2.613, p=0.077). Instead, alterations in axial length, associated with diffuser-wear and topical administration of dopamine, represented changes in vitreal chamber depth (ANOVA, F(3,23)=9.811, p<0.000).

### EXAMPLE 3 - EFFECT OF CO-TREATMENT OF DOPAMINE WITH ATROPINE, PIRENZEPINE AND TPMPA ON FORM DEPRIVATION MYOPIA DEVELOPMENT

70 white male cockerel chickens were randomly assigned to one of 14 treatment groups as defined below (n = 5 per group) and were treated for a four day period.
- Chicks fitted with a translucent diffuser over their left eye to induce FDM;
- Age-matched untreated control group;
- Chicks fitted with a translucent diffuser over their left eye and daily intravitreal injection of a 0.15 mM (0.0028%) dopamine solution prepared according to Example 1.
- Chicks fitted with a translucent diffuser over their left eye and daily intravitreal injection of a 0.25 mM (0.018%) atropine solution;
- Chicks fitted with a translucent diffuser over their left eye and daily intravitreal injection of a 0.15 mM (0.0028%) dopamine and 0.25mM (0.018%) atropine solution prepared according to Example 1;
- Chicks fitted with a translucent diffuser over their left eye and daily intravitreal injection of a 17 mM (0.72%) pirenzepine solution;
- Chicks fitted with a translucent diffuser over their left eye and daily intravitreal injection of a 0.15 mM (0.0028%) dopamine and a 17 mM (0.72%) pirenzepine solution prepared according to Example 1;
- Chicks fitted with a translucent diffuser over their left eye and daily intravitreal injection of an 18.6 mM (0.29%) TPMPA solution;
- Chicks fitted with a translucent diffuser over their left eye and daily intravitreal injection of a 0.15 mM (0.0028%) dopamine and an 18.6 mM (0.29%) TPMPA solution prepared according to Example 1;
- Chicks fitted with a translucent diffuser over their left eye and twice daily topical administration of a 1.5 mM (0.028%) dopamine solution prepared according to Example 1;
- Chicks fitted with a translucent diffuser over their left eye and twice daily topical administration of a 50 mM (3.5%) atropine solution;
- Chicks fitted with a translucent diffuser over their left eye and twice daily topical administration of a 1.5 mM (0.028%) dopamine and a 50 mM (3.5%) atropine solution prepared according to Example 1;
- Chicks fitted with a translucent diffuser over their left eye and twice daily topical administration of an 18.6 mM (0.29%) TPMPA solution;
- Chicks fitted with a translucent diffuser over their left eye and twice daily topical administration of a 1.5 mM (0.028%) dopamine and an 18.6 mM (0.29%) TPMPA solution prepared according to Example 1.

Atropine solutions were prepared by dissolving atropine sulfate monohydrate in a solution containing 0.1% ascorbic acid in 1X PBS to a final concentration of 0.25 mM (0.018% w/v) or 50mM (3.5% w/v), and adjusting the pH to 7. Pirenzepine solutions were prepared by dissolving pirenzepine dihydrochloride in a solution containing 0.1% ascorbic acid in 1X PBS to a final concentration of 17 mM (0.72% w/v), and adjusting the pH to 7. TPMPA solutions were prepared by dissolving TPMPA hydrate in a solution containing 0.1% ascorbic acid in 1X PBS to a final concentration of 18.6 mM (0.29% w/v), and adjusting the pH to 7.

Administration of test compositions and measurement of ocular parameters was performed in accordance with that described in Example 2.

### Results

The results are presented in Figures 2 and 3. Administration of atropine, a muscarinic acetylcholine receptor antagonist; pirenzepine, an M1 muscarinic acetylcholine receptor antagonist; and TPMPA, a GABA_{C} receptor antagonist; either alone or in combination with dopamine (0.15 mM) by intravitreal injection significantly inhibited the excessive axial elongation associated with form-deprivation myopia (ANOVA, F(8,53)=7.894, p<0.000; Figure 2). Importantly, combining dopamine with any of the three compounds elicited a small but significant improvement in the degree to which form-deprivation myopia was inhibited compared to when these compounds were administered alone (dopamine with atropine: 8.76±0.02 mm, p<0.000; dopamine with pirenzepine: 8.76±0.03 mm, p<0.000; dopamine with TPMPA: 8.60±0.07 mm, p<0.000). There was no significant difference in anterior chamber depth (ANOVA, F(8,53)=0.426, p=0.900) or lens thickness (ANOVA, F(8,53)=1.349, p=0.241) across all conditions tested. Instead, alterations in axial length, associated with diffuser-wear and intravitreal injections of test compounds, represented changes in vitreal chamber depth (ANOVA, F(8,53)=7.561, p<0.000).

Topical administration of dopamine (1.5 mM) either alone or in combination with atropine or TPMPA significantly inhibited the excessive axial elongation driven by form-deprivation myopia (ANOVA, F(6,61)=7.357, p<0.000; Figure 3). As neither anterior chamber depth (ANOVA, F(6,61)=0.624, p=0.710), or lens thickness (ANOVA, F(6,61)=1.534, p=0.183) were altered by treatment, changes in axial length represented alterations in vitreal chamber depth (ANOVA, F(6,61)=6.703, p<0.000). As can be seen in Figure 3, the combination of dopamine and atropine (dopamine: 8.97±0.08 mm, p=0.448; atropine: 8.79±0.09 mm, p=0.030; dopamine with atropine: 8.67±0.05 mm, p=0.001), and dopamine and TPMPA (TPMPA: 8.85±0.05 mm, p=0.062; dopamine with TPMPA: 8.69±0.03 mm, p<0.000) significantly increased the degree to which form-deprivation myopia was inhibited compared to each compound alone.

### EXAMPLE 4 - PREPARATION OF DOPAMINE-1,1,2,2-D₄ COMPOSITIONS

To make a 150 mM stock solution, 29 mg dopamine-1,1,2,2-d₄ (in the form of dopamine-1,1,2,2-d₄ hydrochloride, commercially available from Sigma-Aldrich Co. LLC) was dissolved in 1 mL of a solution containing 0.1% ascorbic acid in 1x PBS (pH approximately 5.5). The stock solution was further diluted in an appropriate volume of a solution containing 0.1% ascorbic acid in 1x PBS to generate 0.15 mM (0.0029% w/v), 1.5 mM (0.029% w/v) and 15 mM (0.29% w/v) solutions.

Combination solutions were prepared by adding the appropriate amount of atropine (in the form of atropine sulfate monohydrate, commercially available from Sigma-Aldrich Co. LLC) or TPMPA (in the form of TPMPA hydrate, commercially available from Sigma-Aldrich Co. LLC) to a 1 mL solution of dopamine-1,1,2,2-d₄ prepared above.

### EXAMPLE 5 - EFFECT OF DOPAMINE-1,1,2,2-D₄ ON COMPOSITIONS ON FORM DEPRIVATION MYOPIA DEVELOPMENT

30 white male cockerel chickens were randomly assigned to one of six treatment groups as defined below (n = 5 per group) and were treated for a four day period.
- Chicks fitted with a translucent diffuser over their left eye to induce FDM;
- Chicks fitted with a translucent diffuser over their left eye and daily intravitreal injection of a 15 mM (0.29%) dopamine-1,1,2,2-d₄ solution prepared according to Example 4;
- Chicks fitted with a translucent diffuser over their left eye and daily intravitreal injection of a 1.5 mM (0.029%) dopamine-1,1,2,2-d₄ solution prepared according to Example 4;
- Chicks fitted with a translucent diffuser over their left eye and twice daily topical administration of a 15 mM (0.29%) dopamine-1,1,2,2-d₄ solution prepared according to Example 4;
- Chicks fitted with a translucent diffuser over their left eye and twice daily topical administration of a 1.5 mM (0.029%) dopamine-1,1,2,2-d₄ solution prepared according to Example 4;
- Age-matched untreated control group.

Administration of test compositions and measurement of ocular parameters was performed in accordance with that described in Example 2.

### Results

The results are presented in Figure 4. Intravitreal administration of the dopamine-1,1,2,2-d₄ solution significantly inhibited the axial growth associated with form-deprivation myopia (ANOVA, F(3,22)=13.562, p<0.000; Figure 4). As can be seen in Figure 4, both concentrations of dopamine-1,1,2,2-d₄ tested (1.5 mM and 15 mM) demonstrated a similar level of protection relative to the axial elongation seen in diffuser-only treated animals (1.5 mM: 8.77±0.11 mm, 92% protection, p<0.001; 15 mM: 8.72±0.06 mm, 103% protection, p<0.001). At both concentrations of dopamine-1,1,2,2-d₄, the axial length of diffuser-treated eyes was not different to their age-matched untreated counterparts (1.5 mM p=0.686, 15 mM p=0.707). Across all conditions tested there was no significant difference in both anterior chamber depth (ANOVA, F(3,24)=0.646, p=0.594) and lens thickness (ANOVA, F(3,24)=1.627, p=0.213). Instead, alterations in axial length, associated with diffuser-wear and intravitreal injections of dopamine, represented changes in vitreal chamber depth (ANOVA, F(3,24)=9.592, p<0.000).

Topical application of the dopamine-1,1,2,2-d₄ solution significantly inhibited form-deprivation myopia (ANOVA, F(3,24)=5.346, p=0.006; Figure 4). As with dopamine, the excessive growth associated with form-deprivation myopia was inhibited in a dose-dependent manner by the topical application of dopamine-1,1,2,2-d₄, with a significant effect observed at a concentration of 15 mM (8.85±0.14 mm, 71% protection relative to FDM-only, p<0.01), at which point diffuser-treated eyes were not statistically different in axial length from untreated control eyes (p=0.407). Across all conditions there was no significant difference in both anterior chamber depth (ANOVA, F(3,24)=0.303, p=0.823) and lens thickness (ANOVA, F(3,24)=0.436, p=0.730). Instead, alterations in axial length, associated with diffuser-wear and topical administration of dopamine, represented changes in vitreal chamber depth (ANOVA, F(3,24)=4.379, p=0.015).

### EXAMPLE 6 - EFFECT OF CO-TREATMENT OF DOPAMINE-1,1,2,2-D₄ WITH ATROPINE AND TPMPA ON FORM DEPRIVATION MYOPIA DEVELOPMENT

60 white male cockerel chickens were randomly assigned to one of 12 treatment groups as defined below (n = 5 per group) and were treated for a four day period.
- Chicks fitted with a translucent diffuser over their left eye to induce FDM;
- Age-matched untreated control group;
- Chicks fitted with a translucent diffuser over their left eye and daily intravitreal injection of a 0.15 mM (0.0029%) dopamine-1,1,2,2-d₄ solution prepared according to Example 4;
- Chicks fitted with a translucent diffuser over their left eye and daily intravitreal injection of a 0.25 mM (0.018%) atropine solution;
- Chicks fitted with a translucent diffuser over their left eye and daily intravitreal injection of a 0.15 mM (0.0029%) dopamine-1,1,2,2-d₄ and a 0.25 mM (0.018%) atropine solution prepared according to Example 4;
- Chicks fitted with a translucent diffuser over their left eye and daily intravitreal injection of an 18.6 mM (0.29%) TPMPA solution;
- Chicks fitted with a translucent diffuser over their left eye and daily intravitreal injection of a 0.15 mM (0.0029%) dopamine-1,1,2,2-d₄ and an 18.6 mM (0.29%) TPMPA solution prepared according to Example 4;
- Chicks fitted with a translucent diffuser over their left eye and twice daily topical administration of a 1.5 mM (0.029%) dopamine-1,1,2,2-d₄ solution prepared according to Example 4;
- Chicks fitted with a translucent diffuser over their left eye and twice daily topical administration of a 50 mM (3.5%) atropine solution;
- Chicks fitted with a translucent diffuser over their left eye and twice daily topical administration of a 1.5 mM (0.029%) dopamine-1,1,2,2-d₄ and a 50 mM (3.5%) atropine solution prepared according to Example 4;
- Chicks fitted with a translucent diffuser over their left eye and twice daily topical administration of an 18.6 mM (0.29%) TPMPA solution.
- Chicks fitted with a translucent diffuser over their left eye and twice daily topical administration of a 1.5 mM (0.029%) dopamine-1,1,2,2-d₄ and an 18.6 mM (0.29%) TPMPA solution prepared according to Example 4.

Atropine and TPMPA solutions were prepared in accordance with Example 3. Administration of test compositions and measurement of ocular parameters was performed in accordance with that described in Example 2.

### Results

The results are presented in Figures 5 and 6. Administration of dopamine-1,1,2,2-d₄ combined with either atropine or TPMPA via intravitreal injection, or the administration of each of these compounds alone via intravitreal injection, significantly inhibited the excessive axial elongation associated with form-deprivation myopia (ANOVA, F(6,44)=16.918, p<0.000; Figure 5). Importantly, combining dopamine-1,1,2,2-d₄ with TPMPA elicited a small but significant improvement in the degree to which form-deprivation myopia was inhibited compared to when these compounds were administered alone (dopamine-1,1,2,2-d₄ with TPMPA: 8.61±0.05 mm, p=0.014). Combining dopamine-1,1,2,2-d₄ with atropine produced a small but not significant improvement in the degree to which form-deprivation myopia was inhibited compared to administration of these compounds alone (dopamine-1,1,2,2-d₄ with atropine: 8.73±0.07 mm, p=0.068). There was no significant difference in anterior chamber depth (ANOVA, F(6,44)=0.508, p=0.809) or lens thickness (ANOVA, F(6,44)=0.626, p=0.708) across all conditions. Instead, alterations in axial length, associated with diffuser-wear and intravitreal injections of the test compounds represented changes in vitreal chamber depth (ANOVA, F(6,44)=12.758, p<0.000).

The topical administration of dopamine-1,1,2,2-d₄ (1.5 mM) alone or in combination with atropine or TPMPA significantly inhibited the axial growth associated with form deprivation (ANOVA, F(6,57)=4.616, p=0.001; Figure 6). There was no significant difference in anterior chamber depth (ANOVA, F(6,57)=0.615, p=0.718) or lens thickness (ANOVA, F(6,57)=0.866, p=0.526), rather, alterations in axial length arose from changes in vitreal chamber depth (ANOVA, F(6,57)=5.485, p<0.000). Although increased inhibition of form-deprivation myopia was observed with the combination of dopamine-1,1,2,2-d₄ and TPMPA (dopamine-1,1,2,2-d₄: 8.88±0.10 mm, p=0.105; TPMPA: 8.85±0.09 mm, p=0.062; dopamine-1,1,2,2-d₄ with TPMPA: 8.80±0.03, p=0.015), the same effect was not observed with the combination of dopamine-1,1,2,2-d₄ with atropine (atropine: 8.79±0.09 mm, p=0.030; dopamine-1,1,2,2-d₄ with atropine: 8.80±0.11 mm, p=0.070).

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

## Claims

1. A composition comprising dopamine or a pharmaceutically acceptable salt thereof, or deuterated dopamine or a pharmaceutically acceptable salt thereof, and an aqueous carrier for use in inhibiting the development or progression of a visual disorder of the posterior segment of the eye in a subject, wherein said composition is to be topically administered to an eye of the subject, and wherein said composition is formulated for topical administration to an eye of the subject.

2. The composition for use according to claim 1, wherein the deuterated dopamine is dopamine-1,1,2,2-d₄ [2-(3,4-dihydroxyphenyl)ethyl-1,1,2,2,d₄-amine]; 2-(3,4-dihydroxyphenyl)ethyl-1-deutero-amine; 2-(3,4-dihydroxyphenyl)ethyl-2,2-dideutero-amine; or a pharmaceutically acceptable salt thereof.

3. The composition for use according to claim 1, wherein the deuterated dopamine is dopamine-1,1,2,2-d₄ hydrochloride.

4. The composition for use according to claim 1, wherein the deuterated dopamine or pharmaceutically acceptable salt thereof is a compound of Formula I: 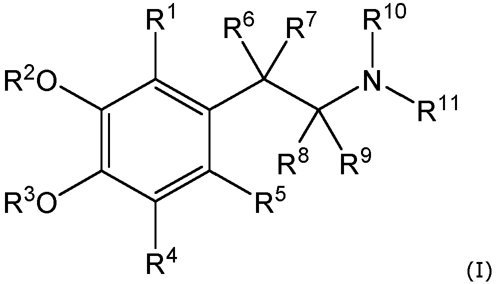
or a pharmaceutically acceptable salt thereof, wherein
R¹, R², R³, R⁴, R⁵, R⁶, R⁷, R⁸, R¹⁰ and R¹¹ are each independently selected from H and D;
R⁹ is selected from H and D ; and
wherein at least one of R¹ to R¹¹ is D.

5. The composition for use according to any one of claims 1-4, wherein the aqueous carrier is selected from the group consisting of saline, water, aqueous buffer, an aqueous solution comprising water and a miscible solvent, and combinations thereof.

6. The composition for use according to any one of claims 1-5, wherein the composition further comprises an antioxidant; preferably wherein the antioxidant is selected from the group consisting of ascorbic acid, phenolic acids, sorbic acid, sodium bisulfite, sodium metabisulfite, acetyl cysteine, sodium thiosulfate, ethylene diamine tetraacetic acid, sodium nitrite, ascorbyl stearate, ascorbyl palmitate, alpha-thioglycerol, erythorbic acid, cysteine hydrochloride, citric acid, tocopherol or vitamin E, tocopherol acetate, dibutylhydroxytoluene, soybean lecithin, sodium thioglycolate, butylhydroxyanisole, propyl gallate, uric acid, melatonin, thiourea, and pharmaceutically acceptable salts and combinations thereof.

7. The composition for use according to any one of claims 1-6, wherein the visual disorder of the posterior segment of the eye is selected from the group consisting of myopia, a visual disorder associated with diabetic retinopathy, and a visual disorder associated with Parkinson's disease.

8. The composition for use according to claim 7, wherein the visual disorder of the posterior segment of the eye is myopia.

9. The composition for use according to any one of claims 1-8, wherein the composition further comprises a dopamine receptor agonist; preferably wherein the dopamine receptor agonist is selected from the group consisting of levodopa, quinpirole, apomorphine, ropinirole, pramipexole, dexpramipexole, piribedil, rotigotine, bromocriptine, lisuride, cabergoline, 2-amino-6,7-dihydroxy-1,2,3,4-tetrahydronaphthalene, pergolide, calidopa, dihydrexidine, doxathrine, propylnorapomorphine, quinagolide, roxindole, sumanirole, fenoldopam, ergocornine, 1-phenyl-2,3,4,5-tetrahydro-(1H)-3-benzazepine-7,8-diol, 2-(N-phenethyl-N-propyl)amino-5-hydroxytetralin, dihydroergotamine, (1R,3S)-1-(aminomethyl)-3-phenyl-3,4-dihydro-1H-isochromene-5,6-diol, carmoxirole, fenoldopam, and pharmaceutically acceptable salts and combinations thereof.

10. The composition for use according to any one of claims 1-9, wherein the composition further comprises a GABA receptor antagonist; preferably wherein the GABA receptor antagonist is selected from the group consisting of bicuculline, flumazenil, gabazine, phenylenetetrazol, (1,2,5,6-tetrahydropyridin-4-yl)methylphosphinic acid, (3-aminopropyl)(cyclohexylmethyl)phosphinic acid, and pharmaceutically acceptable salts and combinations thereof.

11. The composition for use according to any one of claims 1-10, wherein the composition further comprises a muscarinic acetylcholine receptor antagonist; preferably wherein the muscarinic acetylcholine receptor antagonist is selected from the group consisting of atropine, pirenzepine, himbacine, hyoscine, cyclopentolate, ipratropium, oxitropium, tropicamide, oxybutynin, tolterodine, diphenhydramine, dicycloverine, flavoxate, tiotropium, trihexyphenidyl, solifenacin, darifenacin, benzatropine, mebeverine, procyclidine, aclidinium, and pharmaceutically acceptable salts and combinations thereof.

12. The composition for use according to any one of claims 1-11, wherein the composition is formulated for penetration of dopamine or a pharmaceutically acceptable salt thereof through the corneal epithelium.

13. The composition for use according to any one of claims 1-12, wherein the pH of the composition is in the range of from 4 to 8; preferably in the range of from 5.0 to 7.0; more preferably in the range of from 5.5 to 6.5.

## Patentansprüche

1. Zusammensetzung, umfassend Dopamin oder ein pharmazeutisch verträgliches Salz davon oder deuteriertes Dopamin oder ein pharmazeutisches Salz davon sowie einen wässrigen Träger zur Verwendung bei der Hemmung der Entstehung oder des Fortschreitens einer Sehstörung des hinteren Augenabschnitts bei einem Individuum, wobei die Zusammensetzung topisch an ein Auge des Individuums zu verabreichen ist und wobei die Zusammensetzung zur topischen Verabreichung an ein Auge des Individuums formuliert ist.

2. Zusammensetzung zur Verwendung nach Anspruch 1, wobei das deuterierte Dopamin Dopamin-1,1,2,2-d₄[2-(3,4-Dihydroxyphenyl)ethyl-1,1,2,2,d₄-amin]; 2-(3,4-Dihydroxyphenyl)ethyl-1-deutero-amin; 2-(3,4-Dihydroxyphenyl)ethyl-2,2-dideutero-amin oder ein pharmazeutisch verträgliches Salz davon ist.

3. Zusammensetzung zur Verwendung nach Anspruch 1, wobei das deuterierte Dopamin Dopamin-1,1,2,2-d₄-hydrochlorid ist.

4. Zusammensetzung zur Verwendung nach Anspruch 1, wobei das deuterierte Dopamin oder das pharmazeutisch verträgliche Salz davon eine Verbindung der Formel I: 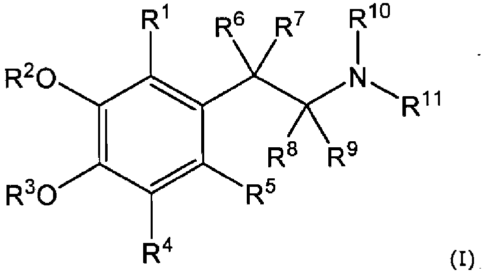
oder ein pharmazeutisch verträgliches Salz davon ist, wobei
R¹, R², R³, R⁴, R⁵, R⁶, R⁷, R⁸, R¹⁰ und R¹¹ jeweils unabhängig voneinander aus H und D ausgewählt sind;
R⁹ aus H und D ausgewählt ist; und
wobei mindestens eines von R¹ bis R¹¹ D ist.

5. Zusammensetzung zur Verwendung nach einem der Ansprüche 1 bis 4, wobei der wässrige Träger ausgewählt ist aus der Gruppe bestehend aus Kochsalzlösung, Wasser, wässrigem Puffer, einer wässrigen Lösung, die Wasser und ein mischbares Lösungsmittel umfasst, sowie Kombinationen davon.

6. Zusammensetzung zur Verwendung nach einem der Ansprüche 1 bis 5, wobei die Zusammensetzung des Weiteren ein Antioxidans umfasst; bevorzugt wobei das Antioxidans ausgewählt ist aus der Gruppe bestehend aus Ascorbinsäure, Phenolsäuren, Sorbinsäure, Natriumbisulfit, Natriummetabisulfit, Acetylcystein, Natriumthiosulfat, Ethylendiamintetraessigsäure, Natriumnitrit, Ascorbylstearat, Ascorbylpalmitat, Alpha-Thioglycerin, Erythorbinsäure, Cysteinhydrochlorid, Zitronensäure, Tocopherol oder Vitamin E, Tocopherolacetat, Dibutylhydroxytoluol, Sojalecithin, Natriumthioglycolat, Butylhydroxyanisol, Propylgallat, Harnsäure, Melatonin, Thioharnstoff und pharmazeutisch verträglichen Salzen und Kombinationen davon.

7. Zusammensetzung zur Verwendung nach einem der Ansprüche 1 bis 6, wobei die Sehstörung des hinteren Augenabschnitts ausgewählt ist aus der Gruppe bestehend aus Kurzsichtigkeit, einer Sehstörung, die mit diabetischer Retinopahtie in Zusammenhang steht, und einer Sehstörung, die mit der Parkinson-Krankheit in Zusammenhang steht.

8. Zusammensetzung zur Verwendung nach Anspruch 7, wobei die Sehstörung des hinteren Augenabschnitts Kurzsichtigkeit ist.

9. Zusammensetzung zur Verwendung nach einem der Ansprüche 1 bis 8, wobei die Zusammensetzung des Weiteren einen Dopaminrezeptoragonisten umfasst, bevorzugt wobei der Dopaminrezeptoragonist ausgewählt ist aus der Gruppe bestehend aus Levodopa, Quinpirol, Apomorphin, Ropinirol, Pramipexol, Dexpramipexol, Piribedil, Rotigotin, Bromocriptin, Lisurid, Cabergolin, 2-Amino-6,7-dihydroxy-1,2,3,4-tetrahydronaphthalin, Pergolid, Calidopa, Dihydrexidin, Doxathrin, Propylnorapomorphin, Quinagolid, Roxindol, Sumanirol, Fenoldopam, Ergocornin, 1-Phenyl-2,3,4,5-tetrahydro-(1H)-3-benzazepin-7,8-diol, 2-(N-Phenethyl-N-propyl)amino-5-hydroxytetralin, Dihydroergotamin, (1R,3S)-1-(Aminomethyl)-3-phenyl-3,4-dihydro-1H-isochromen-5,6-diol, Carmoxirol, Fenoldopam und pharmazeutisch verträglichen Salzen und Kombinationen davon.

10. Zusammensetzung zur Verwendung nach einem der Ansprüche 1 bis 9, wobei die Zusammensetzung des Weiteren einen GABA-Rezeptorantagonisten umfasst; bevorzugt wobei der GABA-Rezeptorantagonist ausgewählt ist aus der Gruppe bestehend aus Bicucullin, Flumazenil, Gabazin, Phenylentetrazol, (1,2,5,6-Tetrahydropyridin-4-yl)methylphosphinsäure, (3-Aminopropyl)(cyclohexylmethyl)phosphinsäure und pharmazeutisch verträglichen Salzen und Kombinationen davon.

11. Zusammensetzung zur Verwendung nach einem der Ansprüche 1 bis 10, wobei die Zusammensetzung des Weiteren einen Muscarin-Acetylcholinrezeptor-Antagonisten umfasst; bevorzugt wobei der Muscarin-Acetylcholinrezeptor-Antagonist ausgewählt ist aus der Gruppe bestehend aus Atropin, Pirenzepin, Himbacin, Hyoscin, Cyclopentolat, Ipratropium, Oxitropium, Tropicamid, Oxybutynin, Tolterodin, Diphenhydramin, Dicycloverin, Flavoxat, Tiotropium, Trihexyphenidyl, Solifenacin, Darifenacin, Benzatropin, Mebeverin, Procyclidin, Aclidinium sowie pharmazeutisch verträglichen Salzen und Kombinationen davon.

12. Zusammensetzung zur Verwendung nach einem der Ansprüche 1 bis 11, wobei die Zusammensetzung so formuliert ist, dass Dopamin oder ein pharmazeutisch verträgliches Salz davon durch das Hornhautepithel dringt.

13. Zusammensetzung zur Verwendung nach einem der Ansprüche 1 bis 12, wobei der pH-Wert der Zusammensetzung im Bereich von 4 bis 8; bevorzugt im Bereich von 5,0 bis 7,0; stärker bevorzugt im Bereich von 5,5 bis 6,5 liegt.

## Revendications

1. Composition comprenant de la dopamine ou un sel pharmaceutiquement acceptable de celle-ci, ou de la dopamine deutérée ou un sel pharmaceutiquement acceptable de celle-ci, et un support aqueux, pour une utilisation dans l'inhibition du développement ou de la progression d'un trouble visuel du segment postérieur de l'œil chez un sujet, dans laquelle ladite composition est destinée à être administrée par voie topique dans un œil du sujet, et dans laquelle ladite composition est formulée pour une administration topique dans un œil du sujet.

2. Composition pour une utilisation selon la revendication 1, dans laquelle la dopamine deutérée est la dopamine-1,1,2,2-d₄ [2-(3,4-dihydroxyphényl)éthyl-1,1,2,2,d₄-amine] ; de la 2-(3,4-dihydroxyphényl)éthyl-1-deutéro-amine ; de la 2-(3,4-dihydroxyphényl)éthyl-2,2-dideutéro-amine ; ou un sel pharmaceutiquement acceptable de celles-ci.

3. Composition pour une utilisation selon la revendication 1, dans laquelle la dopamine deutérée est le chlorhydrate de dopamine-1,1,2,2-d₄.

4. Composition pour une utilisation selon la revendication 1, dans laquelle la dopamine deutérée ou le sel pharmaceutiquement acceptable de celle-ci est un composé de formule I : 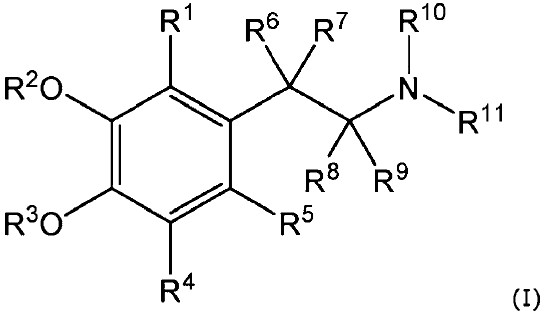
ou un sel pharmaceutiquement acceptable de celui-ci, dans lequel
R¹, R², R³, R⁴, R⁵, R⁶, R⁷, R⁸, R¹⁰ et R¹¹ sont chacun indépendamment choisis parmi H et D ;
R⁹ est choisi parmi H et D ; et
dans lequel au moins un de R¹ à R¹¹ est D.

5. Composition pour une utilisation selon l'une quelconque des revendications 1 à 4, dans laquelle le support aqueux est choisi dans le groupe constitué par une solution saline, de l'eau, un tampon aqueux, une solution aqueuse comprenant de l'eau et un solvant miscible, et des combinaisons de ceux-ci.

6. Composition pour une utilisation selon l'une quelconque des revendications 1 à 5, dans laquelle la composition comprend en outre un antioxydant ; de préférence dans laquelle, l'antioxydant est choisi dans le groupe constitué par l'acide ascorbique, les acides phénoliques, l'acide sorbique, le bisulfite de sodium, le métabisulfite de sodium, l'acétylcystéine, le thiosulfate de sodium, l'acide éthylène diamine tétraacétique, le nitrite de sodium, le stéarate d'ascorbyle, le palmitate d'ascorbyle, l'alpha-thioglycérol, l'acide érythorbique, le chlorhydrate de cystéine, l'acide citrique, le tocophérol ou vitamine E, l'acétate de tocophérol, le dibutylhydroxytoluène, la lécithine de soja, le thioglycolate de sodium, le butylhydroxyanisole, le gallate de propyle, l'acide urique, la mélatonine, la thiourée, et les sels et combinaisons pharmaceutiquement acceptables de ceux-ci.

7. Composition pour une utilisation selon l'une quelconque des revendications 1 à 6, dans laquelle le trouble visuel du segment postérieur de l'œil est choisi dans le groupe constitué par la myopie, un trouble visuel associé à la rétinopathie diabétique et un trouble visuel associé à la maladie de Parkinson.

8. Composition pour une utilisation selon la revendication 7, dans laquelle le trouble visuel du segment postérieur de l'œil est la myopie.

9. Composition pour une utilisation selon l'une quelconque des revendications 1 à 8, dans laquelle la composition comprend en outre un agoniste des récepteurs de la dopamine ; de préférence, dans laquelle l'agoniste des récepteurs de la dopamine est choisi dans le groupe constitué par la lévodopa, le quinpirole, l'apomorphine, le ropinirole, le pramipexole, le dexpramipexole, le piribédil, la rotigotine, la bromocriptine, le lisuride, la cabergoline, le 2-amino-6,7-dihydroxy-1,2,3,4-tétrahydronaphtalène, le pergolide, la calidopa, la dihydrexidine, la doxathrine, la propylnorapomorphine, le quinagolide, le roxindole, le sumanirole, le fénoldopam, l'ergocornine, la 1-phényl-2,3,4,5-tétrahydro-(*1H*)-3-benzazépine-7,8-diol, 2-*(N-phénéthyl-N-propyl*)amino-5-hydroxytétraline, la dihydroergotamine, le (1*R*,3*S*)-1-(aminométhyl)-3-phényl-3,4-dihydro-1*H-*isochromène-5,6-diol, le carmoxirole, le fénoldopam, et les sels et combinaisons pharmaceutiquement acceptables de ceux-ci.

10. Composition pour une utilisation selon l'une quelconque des revendications 1 à 9, dans laquelle la composition comprend en outre un antagoniste des récepteurs GABA ; de préférence dans laquelle l'antagoniste des récepteurs GABA est choisi dans le groupe constitué par la bicuculline, le flumazénil, la gabazine, le phénylènetétrazol, l'acide (1,2,5,6-tétrahydropyridin-4-yl)méthylphosphinique, l'acide (3-aminopropyl)(cyclohexylméthyl)phosphinique, et les sels et combinaisons pharmaceutiquement acceptables de ceux-ci.

11. Composition pour une utilisation selon l'une quelconque des revendications 1 à 10, dans laquelle la composition comprend en outre un antagoniste des récepteurs muscariniques de l'acétylcholine ; de préférence, dans laquelle l'antagoniste des récepteurs muscariniques de l'acétylcholine est choisi dans le groupe constitué par l'atropine, la pirenzépine, l'himbacine, l'hyoscine, le cyclopentolate, l'ipratropium, l'oxitropium, le tropicamide, l'oxybutynine, la toltérodine, la diphénhydramine, la dicyclovérine, le flavoxate, le tiotropium, le trihexyphénidyle, la solifénacine, la darifénacine, la benzatropine, la mébévérine, la procyclidine, l'aclidinium, et les sels et combinaisons pharmaceutiquement acceptables de ceux-ci.

12. Composition pour une utilisation selon l'une quelconque des revendications 1 à 11, dans laquelle la composition est formulée pour la pénétration de la dopamine ou d'un sel pharmaceutiquement acceptable de celle-ci à travers l'épithélium cornéen.

13. Composition pour une utilisation selon l'une quelconque des revendications 1 à 12, dans laquelle le pH de la composition se situe dans la plage de 4 à 8 ; de préférence dans la plage de 5,0 à 7,0 ; plus préférentiellement dans la plage de 5,5 à 6.5.
